# EUROPEAN PATENT APPLICATION

(11) **EP 2 009 022 A1**
(43) Date of publication of application: **31.12.2008**
(21) Application number: 07012523.2
(22) Date of filing: 26.06.2007
(51) Int. Cl.: C07K 14/525, C07K 14/705, C07K 14/715, A61K 47/48, C12N 15/62

(54) **Trimeric death ligands with enhanced activity (tenascin)**

(71) Applicant: Apogenix GmbH, 69120 Heidelberg (DE)
(72) Inventor: The designation of the inventor has not yet been filed
(74) Representative: Weiss, Wolfgang

(57) **Abstract**

The present invention refers to a fusion protein comprising a TNF-superfamily (TNFSF) cytokine or a receptor binding domain thereof fused to a tenascin (TNC) trimerization domain, to a nucleic acid molecule encoding the fusion protein, and to a cell comprising the nucleic acid molecule. The fusion protein is present as a trimeric complex or as an oligomer thereof. The fusion protein, the nucleic acid, and the cell is suitable as pharmaceutical composition or for therapeutic, diagnostic and/or research applications.

## Description

### Field of Invention

The present invention refers to a fusion protein comprising a TNF-superfamily (TNFSF) cytokine or a receptor binding domain thereof fused to a tenascin (TNC) trimerization domain, to a nucleic acid molecule encoding the fusion protein, and to a cell comprising the nucleic acid molecule. The fusion protein is present as a trimeric complex or as an oligomer thereof. The fusion protein, the nucleic acid, and the cell is suitable as pharmaceutical composition or for therapeutic, diagnostic and/or research applications as described herein.

### State of the Art

Ligands of the tumor necrosis factor (TNF) family fulfill crucial roles in the immune system, but have also been implicated in the development of epithelial and endothelial structures.¹ TNF family ligands are primarily expressed as trimeric type II transmembrane proteins and are often processed into soluble variants that are also organized as trimers.^{1,2} While shedding of some TNF ligands does not interfere with their capability to activate their corresponding receptors and might be even important for their physiological function, other TNF ligands become inactivated by proteolytic processing.² Soluble TNF ligands that are not or only poorly active still interact with their cognate receptors. For example, the soluble forms of TNF, CD95L, TRAIL and CD40L interact with TNFR2, CD95, TRAILR2 and CD40, respectively, but do not or only poorly activate signaling by these receptors.³⁻⁶ Notably, inactive or poorly active soluble TNF ligands can be converted into highly active molecules by artificially increasing their avidity. For example, soluble Flag-tagged variants of TNF, CD95L, TRAIL and CD40L stimulate robust signaling by TNFR2, CD95, TRAILR2 and CD40, respectively, provided they were crosslinked with the Flag-specific mAb M2. Likewise, hexameric and dodecameric fusion proteins of soluble CD95L and soluble CD40L as well as non-specifically aggregated preparations of TNF ligands produced in E. coli display high activity.⁶⁻⁸

The structural hall mark of the ligands of the TNF family is the carboxy-terminal "TNF 2 homology domain" or receptor binding domain, both terms are equally used herein, which is part of both the transmembrane and soluble forms of TNF ligands.¹⁻² The THDs of the various TNF ligands are composed of a framework of aromatic and hydrophobic residues that adopt an almost identical tertiary fold and cause self association into trimers.¹⁻² The THD also mediates receptor binding. In general, trimeric ligands of the TNF family bind to three molecules of their corresponding receptor(s). This interaction alone is not necessarily sufficient to activate receptor-associated intracellular signaling pathways. Several lines of evidence suggest that the initial formation of trimeric signaling competent ligand receptor complexes is followed by secondary multimerization into supramolecular clusters.⁹⁻¹¹ These two steps in TNF receptor activation (1. ligand binding; 2. secondary aggregation of receptor ligand complexes) depend to a varying extent on several factors including lipid raft localization, cytoskeleton support, receptor autoaggregation, receptor associated adapter proteins, but also on affinity and avidity of the ligand receptor interaction and the way how the ligand is presented to the receptor (membrane ligand or immobilized ligand versus soluble ligand, trimers versus higher aggregates).

It is known that trimeric complexes of TNF superfamily cytokines are difficult to prepare from recombinant monomeric units.

For example, WO 01/49866 and WO 02/09055 disclose recombinant fusion proteins comprising a TNF cytokine and a multimerization component, particularly a protein from the C1q protein family or a collectin. A disadvantage of these fusion proteins is, however, that the trimerization domain usually has a large molecular weight and/or that the trimerization is rather inefficient.

Schneider et al. (J Exp Med 187 (1989), 1205-1213) describes that trimers of TNF cytokines are stabilized by N-terminally positioned stabilization motifs. In CD95L, the stabilization of the CD95L-receptor binding domain trimer is presumably caused by N-terminal amino acid domains which are located near the cytoplasmic membrane.

Shiraishi et al. (Biochem Biophys Res Commun 322 (2004), 197-202) describes that the receptor binding domain of CD95L may be stabilized by N-terminally positioned artificial α-helical coiled-coil (leucine zipper) motifs. It was found, however, that the orientation of the polypeptide chains to each other, e.g. parallel or antiparallel orientation, can hardly be predicted. Further, the optimal number of hepta-d-repeats in the coiled-coil zipper motif are difficult to determine. In addition, coiled-coil structures have the tendency to form macromolecular aggregates after alteration of pH and/or ionic strength.

Wüest et al. (Oncogene 21 (2002), 4257-4265) describes a TNF-selectokine fusion protein that comprises a single chain antibody targeting module (scFvMO36), a TNC domain, and a TNF domain. Due to the single chain targeting module, the fusion protein is targeted to FAP-positive cells and mimics membrane bound TNF. Thus, the disadvantage of the fusion protein disclosed in Wüest et al. is that their binding and cell killing activity depends on FAP-positive cells or cells adjacent to those cells, respectively.

It was an object of the present invention to provide fusion proteins comprising a TNF cytokine or a receptor binding domain, which allow efficient recombinant manufacture combined with good trimerization properties and improved properties with respect to receptor binding and activation.

### Summary of the Invention

The present invention relates to a fusion protein comprising
(i) a TNF-superfamily cytokine or a receptor binding domain thereof
(ii) a flexible linker element between (i) and.(iii), and
(iii) a tenascin trimerization domain with the provision that in case (iii) is located N-terminally of (i) no scFvMO36-selectokine component is located N-terminally of (iii).

The invention further relates to a nucleic acid molecule encoding a fusion protein as described herein and to a cell or a non-human organism transformed or transfected with a nucleic acid molecule as described herein.

The invention also relates to a pharmaceutical or diagnostic composition comprising as an active agent a fusion protein, a nucleic acid molecule, or a cell as described herein.

The invention also relates to a fusion protein, a nucleic acid molecule, or a cell as described herein for use in therapy, e.g., the use of a fusion protein, a nucleic acid molecule, or a cell as described herein for the preparation of a pharmaceutical composition in the prophylaxis and/or treatment of proliferative disorders, particularly disorders caused by, associated with and/or accompanied by dysfunction of TNF cytokines, such as tumors, e.g. solid or lymphatic tumors, infectious diseases, inflammatory diseases, metabolic diseases, autoimmune disorders, e.g. rheumatoid and/or arthritic diseases, degenerative diseases, e.g. neurodegenerative diseases such as multiple sclerosis, apoptosis-associated diseases and transplant rejections.

### Description of the Figures

**Figure 1**
   Domain architecture of the various CD95L and TRAIL variants used in this study.
   TNC = trimerization domain of chicken tenascin-C (aa 110 - 139), Flag = Flag epitope (DYKDDDDK). The stalk region refers to the structural less defined part between the transmembrane domain and the TNF homology domain of the various ligands.
**Figure 2**
   Soluble variants of mCD95L and mTRAIL encompassing solely the THD are practically inactive. (a) Soluble, N-terminally Flag-tagged variants of hCD95L (Flag-hCD95L(137-281)), mCD95L (Flag-mCD95L(137-279)), hTRAIL (Flag-hTRAIL(95-281)) and mTRAIL (Flag-mTRAIL(99-291)) encompassing the THD were produced in HEK293 cells, purified by M2 affinity chromatography and analyzed by SDS-PAGE and silver staining. Migration positions of molecular mass markers (in kDa) are indicated at the left margin. (b) KB cells (human origin), L929 (murine origin) and mC095 expressing MBL2 transfectants (murine origin) were seeded in triplicates in 96-well plates. The next day, cells (KB, L929) were sensitized for cell death induction by treatment with 2.5 µg/ml CHX and were then stimulated (KB, L929, MBL2-CD95) with the indicated concentrations of Flag-mCD95L(137-279) (KB, MBL2-CD95), Flag-hCD95L(137-281) (KB, MBL2-CD95), Flag-hTRAIL(95-281) (KB, L929) and Flag-mTRAIL(99-291) (KB, L929) in the presence and absence of anti-Flag mAb M2 (CD95L variants: 0.5 µg/ml: TRAIL variants 3 µg/ml). Cell viability was determined by crystal violet staining. (e) Jurkat cells were challenged in triplicates in 96-well plates with the indicated concentration of Flag-hTRAIL(95-281) or M2-crosslinked Flag-hTRAIL(95-281). Cell viability was determined using the MTT assay.
**Figure 3**
   Soluble variants of mCD95L and mTRAIL encompassing the THD do not bind to their corresponding cellular receptors. (a) The indicated cell lines were incubated with varying concentrations of Flag-mCD95L(137-279) (KB, MBL2-CD95), Flag-hCD95L(137-281) (KB, MBL2-CD95), Flag-hTRAIL(95-281) (KB, L929) and Flag-mTRAIL(99-291) (KB,L929) on ice and after repeated washes, bound proteins were detected by FACS using anti-Flag mAb M2 and PE-labelled anti mouse IgG. A soluble N-terminally Flag-tagged variant of human CD40L was used as a control. Left panel shows percent positive gated cells and the right panel show histograms of samples treated with the highest ligand concentration. (b) Cell culture supernatants containing 50 ng of the indicated proteins were analyzed by Western Blotting using primary antibodies specific for mCD95L. (e) KB cells were pretreated for 1 h in triplicates with the indicated reagents. Subsequently, cells were cultured overnight in the presence (black bars) or absence (grey bars) of Fc-hCD95L. Finally, cell viability was determined using the MTT assay (Jurkat) or crystal violet staining (KB). (d) The indicated CD95L variants were analyzed by surface plasmon resonance using BlAcore 2000 and a streptavidin sensor chip coated with biotinylated Fc-CD95. Sensorgrams for binding of the mCD95L variants are shown for concentrations of 5000, 2000, 1000, 500, 300, 200, 100, 30, and 5 nM. Flag-hCD95L sensorgrams are shown for concentrations of 1000, 400, 200,100, 60, 40, 20, 6 and 2 nM. The indicated values for k_{off} and kₒₙ were determined using a 1:1 binding model and the software supplied by the manufacturer for 1000 and were averaged from the plots between 1000 and 20 nM (hCD95L) and 2000 and 30 nM (murine CD95L variants).
**Figure 4**
   Soluble variants of CD95L and TRAIL of human and murine origin as well as their corresponding TNC fusion proteins assemble into trimers. (a) The indicated proteins were treated with the increasing concentrations of the chemical crosslinker BS3 on ice for 1 h and were analyzed under reducing conditions by Western Blot using the anti-Flag mAb M2. Migration positions of molecular mass markers (in kDa) are indicated at the left margin. (b) The indicated proteins were separated by gel filtration chromatography using a BioSep-SEC-S3000 (upper panel) or a BioSep-Sec-S2000 column. The following molecular mass markers were used: thyroglobulin (669 kDa), apoferritin (443 kDa), β-amylase (200 kDa), serum albumin (66 kDa), carbonic anhydrase (29 kDa) and cytochrom c (12.4 kDa).
**Figure 5**
   The stalk region of murine CD95L but not of murine TRAIL is sufficient to convert the corresponding soluble ligands into an active variant. (a-e) KB cells were seeded in triplicates in 96-well plates. The following day, cells were stimulated with the indicated concentrations of M2-crosslinked or non-crosslinked Flag-mCD95L(WX1),(a), Flag-hCD95L(S1),(b), Flag-m/hCD95L (c) and Flag-mTRAIL(43-291) (d) or with a 1:5 dilution of membrane mTRAIL containing lysates (e) in the presence of 2.5 µg/ml CHX. After 18 h, cell viability was determined by crystal violet staining. To demonstrate that membrane TRAIL expressing cells undergo caspase-mediated cell death, the caspase inhibitor Z-Val-Ala-DL-Asp-fluoromethylketone was added (20 µM). The cytotoxic effect of Flag-Flag-hCD95L(137-281) and M2-crosslinked Flag-hCD95L(137-281) was determined in parallel as a control and plotted in "a" to "c" (dotted lines, crosses: no crosslinking; asterisks: M2-crosslinking).
**Figure 6**
   TNC fusion proteins of the THD of mCD95L and mTRAIL interact with their corresponding receptors and stimulate CD95 and TRAILR2 after secondary crosslinking. (a) N-terminally Flag-tagged soluble TNC fusion proteins of mCD95L (Flag-TNC-mCD95L(137-279) and mTRAIL (Flag-TNC-mTRAIL (99-291) and their corresponding "TNC-less" variants were produced in HEK293 cells, purified by M2 affinity chromatography and analyzed under reducing and non-reducing conditions by SDS-PAGE and silver staining. (b) The indicated cell lines were incubated with varying concentrations of Flag-mCD95L(137-279), Flag-TNC-mCD95L(137-279) (KB, MBL2-CD95), Flag-mTRAIL(99-291) and Flag-TNC-mTRAIL(99-291) (KB, L929) on ice and after repeated washes, bound proteins were detected by FACS using anti-Flag mAb M2 and PE-labelled anti mouse IgG. A soluble N-terminally Flag-tagged variant of human CD40L served as a control. (c) KB, L929, and MBL2-mCD95 cells were seeded in triplicates in 96-well plates. The next day, cells (KB and L929) were treated with 2.5 µg/ml CHX and were challenged (KB, L929, and MBL2-mCD95) with the indicated concentrations of Flag-mCD95L(137-279), Flag-TNC-mCD95L(137-279) (KB, MBL2-CD95 Flag-mTRAIL(99-291) (KB, L929) and Flag-TNC-mCD95L(137-279) (KB, L929) in the presence and absence of anti-Flag mAb M2 (CD95L variants: 0.5 µg/ml: TRAIL variants 3 µg/ml). After 18 h cell viability was determined by crystal violet staining. (d) MBL2-mCD95 and L929 cells were challenged in triplicates as indicated with Flag-linker, Flag and Flag-TNC variants of murine CD95L and TRAIL in the presence (grey bars) or absence (black bars) of anti-Flag mAb M2. After overnight incubation, cell viability was determined by crystal violet staining (KB). L929 cells were treated in the presence of 2.5 µg/ml CHX.
**Figure 7**
   TNC fusion proteins of soluble human CD95L and human TRAIL display improved receptor binding and higher activity after secondary crosslinking. (a) N-terminally Flag-tagged soluble TNC fusion proteins of hCD95L (Flag-TNC-hCD95L(137-281) and hTRAIL (Flag-TNC-hTRAIL (95-281) and their "TNC-less" counterparts were produced in HEK293 cells, purified by M2 affinity chromatography and analyzed under reducing and non-reducing conditions by SDS-PAGE and silver staining. (b) The indicated cell lines were incubated with varying concentrations of Flag-hCD95L(137-281), Flag-TNC-hCD95L (137-281) (KB, MBL2-CD95), Flag-hTRAIL (95-281) and Flag-TNC-hTRAIL (95-281) (KB, L929) on ice and after repeated washing, bound proteins were detected by FACS using anti-Flag mAb M2 and PE-labelled anti mouse IgG. (c) KB, L929, and MBL2-mCD95 cells were seeded in triplicates in 96-well plates. The next day, the MBL2-CD95 cells and CHX sensitized KB and L929 cells were stimulated for 18 h with the indicated concentrations of Flag-hCD95L(137-281), Flag-TNC-hCD95L(137-281) (KB, MBL2-CD95), Flag-hTRAIL(95-281) (KB, L929) and Flag-TNC-hTRAIL(95-281) (KB, L929) in the presence and absence of anti-Flag mAb M2 (0.5 µg/ml). Cell viability was finally determined by crystal violet staining. (d) Jurkat cells were challenged in triplicates in 96-well plates with the indicated concentration of Flag-TNC-hTRAIL(95-281) or M2-crosslinked Flag-TNC-hTRAIL(95-281). Cell viability was determined, using the MTT assay. (e) KB cells were sensitized for apoptosis induction by treatment with 2.5 µg/ml CHX for 1 h and were then stimulated for an additional hour with the indicated concentration of M2-crosslinked Flag-TNC-hTRAIL(95-281) or Flag-hTRAIL(95-281). The DISC was then precipitated using protein G sepharose. The precipitates as well as total cell lysates were analyzed for the presence of indicated proteins by Western Blotting. Lysates of untreated cells supplemented with a mixture of 10 ng of the corresponding ligand and 50 ng M2 were immunoprecipitated as controls and are indicated with "-". Due to the excess of M2 antibody used in the control lysates, crossreactivity with the heavy chain part of the molecule is observable in the control lanes of some Western Blots.

### Detailed Description of the Invention

The fusion protein may be a monomeric protein or a multimeric protein. Preferably, the fusion protein is present as a trimeric complex consisting of three monomeric units which may be identical or different. Preferably, a trimeric complex consists of three identical fusion proteins. In a further preferred embodiment, the complex is formed by covalent linkage between three of the fusion proteins described herein, e.g., a covalent linkage of disulfide bridges between two conserved cysteines of the tenacsin trimerization domain(iii) as described herein. The trimeric complex as such shows biological activity. It was found, however, that oligomers of the trimeric complex, e.g. defined complexes wherein the basic trimeric structure is present 2, 3 or 4 times, also have biological activity.

One component (i) of the fusion protein is a cytokine of the TNF superfamily or a receptor binding domain thereof. Preferably, component (i) is a mammalian, particularly human cytokine or a receptor binding domain thereof including allelic variants and/or derivatives thereof. Further, it is preferred that the TNF cytokine is a receptor binding domain thereof capable of binding to the corresponding cytokine receptor and preferably capable of receptor activation, whereby apoptotic or proliferative activity may be caused. The cytokine may e.g. be selected from TNF superfamily members, e.g. human TNFSF-1 to -18 as indicated in Table 2, preferably from CD95L (FasL), TRAIL, TNFα, LTB, CD40L, CD30L, OX40L, RANKL, TWEAK, Lta, Ltab2, LIGHT, CD27L, 41-BB, GITRL, APRIL, EDA, VEGI and BAFF or a receptor binding domain thereof. Preferred receptor binding domains of the respective proteins are indicated in Table 1 (NH₂-aa to COOH-aa).

More preferably, the cytokine of the TNF superfamily or a receptor binding domain thereof is selected from CD95L, TRAIL or TNFα or a receptor binding domain thereof. In an especially preferred embodiment, the cytokine of the TNF superfamily or a receptor binding domain thereof comprises the extracellular portion of a TNF cytokine including the receptor binding domain without membrane located domains.

In an especially preferred embodiment, the cytokine of the TNF superfamily or a receptor binding domain thereof of the fusion protein is selected from human CD95L (SEQ ID NO:12), particularly amino acids 120-281, 137-281, 142-281 or 144-281 of human CD95L or amino acids 137-279 of mouse CD95L (SEQ ID NO:13).

In another especially preferred embodiment, the cytokine of the TNF superfamily or a receptor binding domain thereof of the fusion protein is selected from human TRAIL (SEQ ID NO:14), particularly amino acids 95-281, 116-281, 118-281 or 120-281 of human TRAIL or amino acids 99-291 of mouse TRAIL (SEQ ID NO:15). In another preferred embodiment human TRAIL comprise any amino acid from 95-120 as initial amino acid - amino acid 281 of SEQ ID NO:14.

In a further preferred embodiment of the invention, the cytokine of the TNF superfamily or a receptor binding domain thereof of the fusion protein comprises a mutant of the cytokine of the TNF superfamily or a receptor binding domain thereof which binds and/or activates TRAIL-receptor 1 (TRAILR1) and/or TRAIL-receptor 2 (TRAILR2). The binding and/or activity of the mutant may be, e.g., determined by the assays as disclosed herein, e.g., in the Examples or by the assays disclosed in van der Sloot et al. (PNAS, 2006, 103:8634-8639), Kelley et al. (J. Biol. Chem., 2005, 280:2205-2215), or MacFarlane et al. (Cancer Res., 2005, 65: 11265-11270).

The mutant may be generated by any technique and is known by the skilled person, e.g., the techniques disclosed in an der Sloot et al. (PNAS, 2006, 103:8634-8639), Kelley et al. (J. Biol. Chem., 2005, 280:2205-2215), or MacFarlane et al. (Cancer Res., 2005, 65: 11265-11270) any may comprise any type of structural mutations, e.g., substitution, deletion, duplication and/or insertion of an amino acid. A preferred embodiment is the generation of substitutions. The substitution may affect at least one amino acid of the cytokine of the TNF superfamily or a receptor binding domain thereof. In a preferred embodiment, the substitution may affect at least one of the amino acids of TRAIL, e.g., human TRAIL (e.g., SEQ ID NO:14). Preferred substitutions in this regard affect at least one of the following amino acids of human TRAIL of SEQ ID NO:14: R130, G160, Y189, R191, Q193, E195, N199, K201, Y213, T214, S215, H264, 1266, D267, D269. Preferred-amino acid substitutions of human TRAIL of SEQ ID NO:14 are at least one of the following substitutions: R130E, G160M, Y189A, Y189Q, R191K, Q193S, Q193R, E195R, N199V, N199R, K201R, Y213W, T214R, S215D, H264R, I266L, D267Q, D269H, D269R, or D269K.

The amino acid substitution(s) may affect the binding and/or activity of TRAIL, e.g., human TRAIL, to or on either the TRAILR1 or the TRAILR2. Alternatively, the amino acid substitution(s) may affect the binding and/or activity of TRAIL, e.g., human TRAIL, to or on both, the TRAILR1 and the TRAILR2. The binding and/or activity of the TRAILR1 and/or TRAILR2 may be affected positively, i.e., stronger, more selective or specific binding and/or more activation of the receptor. Alternatively, the binding and/or activity of the TRAILR1 and/or TRAILR2 may be affected negatively, i.e., weaker, less selective or specific binding and/or less or no activation of the receptor.

Examples of mutants of TRAIL with amino acid substitution(s) that affect binding and/or activity of both TRAILR1 and TRAILR2 may be found, e.g., in Table 1 of MacFarlane et al. (cf. above) and may comprise human TRAIL mutants with the following two amino acid substitutions of SEQ ID NO:14 Y213W and S215D or the following single amino acid substitution Y189A.

Examples of mutants of TRAIL with amino acid substitution(s) that affect binding and/or activity of TRAILR1 may be found, e.g., in Table 1 of MacFarlane et al. (cf. above) and may comprise human TRAIL mutants with the following four amino acid substitutions of SEQ ID NO:14 N199V, K201R, Y213W and S215D or the following five amino acid substitutions Q193S, N199V, K201R, Y213W and S215D or in Table 2 of Kelley et al. (cf. above) and may comprise human TRAIL mutants with the following six amino acid substitutions Y213W, S215D, Y189A, Q193S, N199V, and K201R or Y213W, S215D, Y189A, Q193S, N199R, and K201R.

Examples of mutants of TRAIL with amino acid substitution(s) that affect binding and/or activity of TRAILR2 may be found, e.g., in Table 1 of MacFarlane et al. (cf. above) or in Table 2 of Kelley et al. (cf. above) and may comprise human TRAIL mutants with the following six amino acid substitutions of SEQ ID NO:14 Y189Q, R191K, Q193R, H264R, I266L, and D267Q or in Table 2 of van der Sloot et al. (cf. above) and may comprise human TRAIL mutants with the following single amino acid substitution D269H, the following two amino acid substitutions D269H and E195R or D269H and T214R.

A flexible linker element (ii) is located between the cytokine of the TNF superfamily or a receptor binding domain thereof (i) and the tenascin trimerization domain as described herein (iii). The flexible linker element preferably has a length of 3-20 amino acids, particularly a length of 3, 6, 9, 10, 12, 15 or 18 amino acids. The linker element is preferably a glycine/serine linker, i.e., a peptide linker substantially consisting of the amino acids glycine and serine. In an especially preferred embodiment, the linker has the amino acid sequence (GSS)ₐ(SSG)_{b}(GSG)_{c} wherein a, b, c is each 0, 1, 2, 3, 4 or 5. In another preferred embodiment; the linker has the amino acid sequence GTGGGSGGRG. In another preferred embodiment, the linker has the amino acid sequence (GSS)ₙGSG, (SGG)ₙGSG or (GSS)ₙ wherein n=1, 2, 3, 4, or 5.

The tenascin trimerization domain (iii) with the provision that in case the tenascin trimerization domain as described herein is located N-terminally of the cytokine of the TNF superfamily or a receptor binding domain thereof as described herein no scFvMO36-selectokine component is located N-terminally of the tencascin domain may be from different species, e.g., from chicken or human. In a preferred embodiment, the tenascin trimerization domain comprises the amino acid sequence ACGCAAAPDVKELLSRLEELENLVSSLREQ (SEQ ID NO:9) or a sequence having an identity of at least 70% thereto.

Further, it is preferred that the tenascin trimerization domain has a length of from 20 up to 40 amino acids.

In the fusion protein of the invention, it is preferred that the tenascin trimerization domain (iii) is located N-terminally of the cytokine of the TNF superfamily or a receptor binding domain thereof (i) with the provision that no scFvM036-selectokine component is located N-terminally of the tencascin domain.

The invention, however, also refers to embodiments, wherein the cytokine of the TNF superfamily or a receptor binding domain thereof as described herein is located N-terminally of the tenascin trimerization domain (iii).

Another preferred embodiment is a fusion protein wherein the cytokine of the TNF superfamily or a receptor binding domain thereof is TRAIL, particularly human or mouse TRAIL, or a receptor binding domain thereof, preferably amino acids 95-281, 116-281, 118-281 or 120-281 of human TRAIL (SEQ ID NO:14) or amino acids 99-291 of mouse TRAIL (SEQ ID NO:15) and is located N-terminally of the tenascin trimerization domain (iii). Preferred fusion proteins of the invention in this regard comprise the sequences SEQ ID NO:17 or SEQ ID NO:18.

Another preferred embodiment is a fusion protein wherein the cytokine of the TNF superfamily or a receptor binding domain thereof is CD95L, particularly human or mouse CD95L, or a receptor binding domain thereof, preferably amino acids amino acids 120-281, 137-281, 142-281 or 144-281 of human CD95L or amino acids 137-279 of mouse CD95L (SEQ ID NO:13) and is located N-terminally of the tenascin trimerization domain (iii)..

The fusion protein may additionally comprise an N-terminal signal peptide domain, which allows processing, e.g., extracellular secretion, in a suitable host cell. Preferably, the N-terminal signal peptide domain comprises a protease, e.g., a signal peptidase cleavage site and thus may be removed after or during expression to obtain the mature protein. In a preferred embodiment, the N-terminal signal peptide domain comprises the sequence SEQ ID NO:10, SEQ ID NO:11 or SEQ ID NO:16.

Preferred fusion proteins of the invention comprise the sequences SEQ ID NO:1, SEQ ID NO:2, SEQ ID NO:3, SEQ ID NO:4, SEQ ID NO:5, SEQ ID NO:6, SEQ ID NO:7 or SEQ ID NO:8.

Further, the fusion protein may further comprise comprises a recognition/purification domain, e.g., a FLAG domain, a Strep-tag domain and/or a poly-His domain, which may be located at the N-terminus or at the C-terminus.

The fusion protein may additionally comprise a C-terminal flexible element, having a length of, e.g., 1-50, preferably 10-30 amino acids which may include and/or connect to a recognition/purification domain as described herein.

A further aspect of the present invention relates to a nucleic acid molecule encoding a fusion protein as described herein. The nucleic acid molecule may be a DNA molecule, e.g., a double-stranded or single-stranded DNA molecule, or an RNA molecule. The nucleic acid molecule may encode the fusion protein or a precursor thereof, e.g., a pro- or pre-proform of the fusion protein which may comprise a signal sequence as described herein or other heterologous amino acid portions for secretion or purification which are preferably located at the N- and/or C-terminus of the fusion protein as described herein. The heterologous amino acid portions may be linked to the first and/or second domain via a protease cleavage site, e.g., a Factor Xₐ, thrombin or IgA protease cleavage site.

The nucleic acid molecule may be operatively linked to an expression control sequence, e.g. an expression control sequence which allows expression of the nucleic acid molecule in a desired host cell. The nucleic acid molecule may be located on a vector, e.g. a plasmid, a bacteriophage, a viral vector, a chromosal integration vector, etc. Examples of suitable expression control sequences and vectors are described for example by Sambrook et al. (1989) Molecular Cloning, A Laboratory Manual, Cold Spring Harbor Press, and Ausubel et al. (1989), Current Protocols in Molecular Biology, John Wiley & Sons or more recent editions thereof.

Various expression vector/host cell systems may be used to express the nucleic acid sequences encoding the fusion proteins of the present invention. Suitable host cells include, but are not limited to, prokaryotic cells such as bacteria, e.g. *E.coli,* eukaryotic host cells such as yeast cells, insect cells, plant cells or animal cells, preferably mammalian cells and, more preferably, human cells.

Further, the invention relates to a non-human organism, e.g., mouse or rat, transformed or transfected with a nucleic acid molecule as described herein. Such organisms may be comprise knock-out organisms, generated by known methods of genetic transfer including homologous recombination. Alternatively, such organisms may comprise transgenic organisms which comprise several copies of the nucleic acid molecule as described herein. The generation of transgenic organisms is known in the art.

The fusion protein, the nucleic acid coding therefore, the transformed or transfected cell as well as the trimeric complexes or oligomers of the trimeric complexes, all as described herein may be used for pharmaceutical, diagnostic and/or research applications.

A further aspect of the present invention relates to a pharmaceutical or diagnostic composition comprising as an active agent at least one fusion protein, the nucleic acid coding therefore, the transformed or transfected cell as well as the trimeric complexes or oligomers of the trimeric complexes, all as described herein.

At least one fusion protein, the nucleic acid coding therefore, the transformed or transfected cell as well as the trimeric complexes or oligomers of the trimeric complexes, all as described herein may be used in therapy, e.g., in the prophylaxis and/or treatment of disorders selected from proliferative disorders, particularly disorders caused by, associated with and/or accompanied by dysfunction of TNF cytokines, such as tumors, e.g. solid or lymphatic tumors, infectious diseases, inflammatory diseases, metabolic diseases, autoimmune disorders, e.g. rheumatoid and/or arthritic diseases, degenerative diseases, e.g. neurodegenerative diseases such as multiple sclerosis, apoptosis-associated diseases and transplant rejections.

The composition may be administered as monotherapy or as combination therapy with further medicaments, e.g. cytostatic or chemotherapeutic agents, corticosteroids and/or antibiotics.

The fusion protein is administered to a subject in need thereof, particularly a human patient, in a sufficient dose for the treatment of the specific conditions by suitable means. For example, the fusion protein may be formulated as a pharmaceutical composition together with pharmaceutically acceptable carriers, diluents and/or adjuvants. Therapeutic efficacy and toxicity may be determined according to standard protocols. The pharmaceutical composition may be administered systemically, e.g. intraperitoneally, intramuscularly or intravenously or locally, e.g. intranasally, subcutaneously or intrathecally. Preferred is intravenous administration.

The dose of the fusion protein administered will of course be dependent on the subject to be treated, on the subject's weight, the type and severity of the disease, the manner of administration and the judgement of the prescribing physician. For the administration of fusion proteins, a daily dose of 0.001 to 100 mg/kg is suitable.

### References

1. Locksley RM, Killeen N and Lenardo MJ (2001) Cell 104: 487-501
2. Bodmer JL, Schneider P and Tschopp J (2002) Trends Biochem. Sci. 27: 19-26
3. Grell M, Douni E, Wajant H, Lohden M., Clauss M, Maxeiner B, Georgopoulos S, Lesslauer W, Kollias G, Pfizenmaier K and Scheurich P (1995) Cell 83: 793-802
4. Schneider P, Holler N, Bodmer JL, Hahne M, Frei K, Fontana A and Tschopp J (1998) J. Exp. Med. 187: 1205-1213
5. Wajant H, Moosmayer D, Wuest T, Bartke T, Gerlach E, Schonherr U, Peters N, Scheurich P and Pfizenmaier K (2001) Oncogene 20: 4101-4106
6. Haswell LE, Glennie MJ and Al-Shamkhani A (2001) Eur. J. Immunol. 31: 3094-31008
7. Holler N, Tardivel A, Kovacsovics-Bankowski M, Hertig S, Gaide 0, Martinon F, Tinel A, Deperthes D, Calderara S, Schulthess T, Engel J, Schneider P and Tschopp J (2003) Mol. Cell. Biol. 23: 1428-1440
8. Stone GW, Barzee S, Snarsky V, Kee K, Spina CA, Yu XF and Kornbluth RS (2006) J. Virol. 80: 1762-177216
9. Mundle SD and Raza A (2002) Trends Immunol. 23: 187-194
10. Siegel RM, Muppidi JR, Sarker M, Lobito A, Jen M, Martin D, Straus SE and Lenardo MJ (2004) J. Cell Biol. 167: 735-744
11. Henkler F, Behrle E, Dennehy KM, Wicovsky A, Peters N, Warnke C, Pfizenmaier K and Wajant H (2005) J. Cell Biol. 168: 1087-1098
12. Schneider P, Bodmer JL, Holler, Mattmann C, Scuderi P, Tersikikh A, Peitsch MC, Tschopp J (1997) J. Biol. Chem. 272: 18827-18833
13. Muhlenbeck F, Schneider P, Bodmer JL, Schwenzer R, Hauser A, Schubert G, Scheurich P, Moosmayer D, Tschopp J and Wajant H (2000) J. Biol. Chem. 275: 32208-32213
14. Kelley RF, Totpal K, Lindstrom SH, Mathieu M, Billeci K, Deforge L, Pai R, Hymowitz S G and Ashkenazi A (2005) J. Biol. Chern. 280: 2205-2212
15. Wu GS, Burns TF, Zhan Y, Alnemri ES and El-Deiry WS (1999) Cancer Res. 59: 2770-2775
16. Schneider P, Olson D, Tardivel A, Browning B, Lugovskoy A, Gong D, Dobles M, Hertig S, Hofmann K, Van Vlijrnen H, Hsu YM, Burkly LC, Tschopp J and Zheng TS (2003) J.Biol. Chem. 278: 5444-5454
17. Suda T, Tanaka M, Miwa K and Nagata S (1996) J. Immunol. 157: 3918-3924
18. Kammerer RA, Schulthess T, Landwehr R, Lustig A, Fischer D and Engel J. (1998) J: Biol. Chem. 273: 10602-10608
19. Clancy L, Mruk K, Archer K, Woelfel M, Mongkolsapaya J, Screaton G, Lenardo MJ and Chan FK. (2005) PNAS 102:18099-18104
20. Merino D, Lalaoui N, Morizot A, Schneider P, Solary E and Micheau 0 (2006) Mol. Cell.Biol. 26:7046-55
21. Schneider P, Thome M, Burns K, Bodmer JL, Hofmann K, Kataoka T, Holler N and Tschopp J (1997) Immunity 7: 831-836
22. Medema JP, de Jong J, van Hall T, Melief CJ and Offringa R (1999) J. Exp. Med. 190:1033-1038
23. Siegmund 0, Klose S, Zhou 0, Baumann B, Räder C, Kalthoff H, Wajant Hand Trauzold (2007) Cell Signal. 19: 1172-84
24. Shen BJ, Hage T and Sebald W (1995) Eur. J. Biochem. 240: 252-261

### Basic Structure of a Fusion Protein

In the following, the basic structure of the recombinant proteins of the invention is shown exemplified for the receptor binding domain of TRAIL and CD95L.

### 1.1 Sequences of the Signal Peptides

MNFGFSLIFLVLVLKGVQC (SEQ ID NO:10)
METDTLLLWVLLLWVPGSTG (SEQ ID NO:11)
METDTLLLWVLLLWVPAGNG (SEQ ID NO:16)

### 1.2 Flag-epitope/enterokinase-processing site

DYKDDDDKD

### 1.3 Human Tenascin Trimerization Domain

Ala110-Gln139 from P24821
ACGCAAAPDVKELLSRLEELENLVSSLREQ (SEQ ID NO:9)
LOCUS P24821 2201 aa linear PRI 15-MAY-2007
DEFINITION Tenascin precursor (TN) (Tenascin-C) (TN-C) (Hexabrachion) (Cytotactin) (Neuronectin) (GMEM) (JI) (Myotendinous antigen) (Glioma-associated-extracellular matrix antigen) (GP 150-225).
ACCESSION P24821

### 1.4 Flexible Linker Element

(GSS)ₙGSG or (GSS)ₙ or (SGG)ₙGSG or (GSS)ₐ(SSG)_{b}(GSG)_{c} wherein a, b, c, and n are defined as described herein

### 1.5 TNF Receptor Binding Domain

Various fragments of full-length human and mouse CD95L as well as human and mouse TRAIL are conceivable:
a) human CD95L (SEQ iD NO:12) 
1 mqqpfnypyp qiywvdssas spwappgtvl pcptsvprrp gqrrpppppp
ppplpppppp
61 pplpplplpp lkkrgnhstg lcllvmffmv lvalvglglg mfqlfhlqke
laelrestsq
121 mhtasslekq ighpspppek kelrkvahlt gksnsrsmpl ewedtygivl
lsgvkykkgg
181 lvinetglyf vyskvyfrgq scnnlplshk vymrnskypq dlvmmegkmm
syctttgqmwa
241 rssylgavfn ltsadhlyvn vselslvnfe esqtffglyk l
b) murine CD95L (SEQ ID NO:13) 
1 mqqpmnypcp qifwvdssat sswappgsvf pcpscgprgp dqrrpppppp
pvsplpppsq
61 plplppltpl kkkdhntnlw lpvvffmvlv alvgmglgmy qlfhlqkela
elreftnqsl
121 kvssfekqia npstpsekke prsvahltgn phsrsiplew edtygtalis
gvkykkgglv
181 inetglyfvy skvyfrgqsc nnqplnhkvy mrnskypedl vlmeekrlny
cttgqiwahs
241 shlgavfnlt sadhlyvnis qlslinfees ktffglykl
c) human TRAIL (SEQ ID NO:14) 
1 

61 

121 

181 

241 
d) mouse TRAIL (SEQ ID NO:15)

1 mpssgalkdl sfsqhfrmmv icivllqvll qavsvavtym yftnemkqlq
dnyskiglac
61 fsktdedfwd stdgeilnrp clqvkrqlyq lieevtlrtf qdtistvpek
qlstpplprg
121 grpqkvaahi tgitrrsnsa lipiskdgkt lgqkieswes srkghsflnh
vlfrngelvi
181 eqeglyyiys qtyfrfqeae daskmvskdk vrtkqlvqyi ykytsypdpi
vlmksarnsc
241 wsrdaeygly siyqgglfel kkndrifvsv tnehlmdldq easffgafli n

Preferred fragments of human TRAIL start at each amino acid between Thr95 and Gln120 of human TRAIL (SEQ ID NO:14) and terminating at Gly281 of human TRAIL (SEQ ID NO:14), e.g.,
Thr95-Gly281
Glu116- Gly281
Gly118- Gly281
Gln120- Gly281

TSEETISTVQEKQQNISPLVRERGPQRVAAHITGTRGRSNTLSSPNSKNEKALGRKI
NSWESSRSGHSFLSNLHLRNGELVIHEKGFYYIYSQTYFRFQEEIKENTKNDKQMVQ
YIYKYTSYPDPILLMKSARNSCWSKDAEYGLYSIYQGGIFELKENDRIFVSVTNEHL
IDMDHEASFFGAFLVG.

### 1.6 Examples of Fusion Proteins

SEQ ID NO:1          Flag-TNC-hCD95L aa137-281.PRO
KEYWORD      PROTEIN

ORIGIN
1          MNFGFSLIFL  VLVLKGVQCE  VKLVPRGSDY  KDDDDKDIAC  GCAAAPDIKD
LLSRLEELEG
61         LVSSLREQGT  GGGSGGRGEF  PPEKKELRKV  AHLTGKSNSR  SMPLEWEDTY
GIVLLSGVKY
121        KKGGLVINET  GLYFVYSKVY  FRGQSCNNLP  LSHKVYMRNS  KYPQDLVMME
GKMMSYCTTG
181       QMWARSSYLG  AVFNLTSADH  LYVNVSELSL  VNFEESQTFF  GLYKL

SEQ ID NO:2          Flag-TNC-mCD95L aa137-279.PRO
KEYWORD      PROTEIN

ORIGIN
1          MNFGFSLIFL  VLVLKGVQCE  VKLVPRGSDY  KDDDDKDIAC  GCAAAPDIKD
LLSRLEELEG
61         LVSSLREQGT  GGGSGGRGEF  EKKEPRSVAH  LTGNPHSRSI  PLEWEDTYGT
ALISGVKYKK
121        GGLVINETGL  YFVYSKVYFR  GQSCNNQPLN  HKVYMRNSKY  PEDLVLMEEK

RLNYCTTGQI
181 WAHSSYLGAV FNLTSADHLY VNISQLSLIN FEESKTFFGL YKL

SEQ ID NO:3          Flag-TNC-mTRAIL aa99-291.PRO
KEYWORD     PROTEIN

ORIGIN
1          MNFGFSLIFL  VLVLKGVQCE  VKLVPRGSDY  KDDDDKDIAC  GCAAAPDIKD
LLSRLEELEG
61         LVSSLREQGT  GGGSGGRGEF  TFQDTISTVP  EKQLSTPPLP  RGGRPQKVAA
HITGITRRSN
121        SALIPISKDG  KTLGQKIESW  ESLIPISKDG  KTLGQKIESW  ESSRKGHSFL
NHVLFRNGEL
181        VIEQEGLYYI  YSQTYFRFQE  AEDASKMVSK  DKVRTKQLVQ  YIYKYTSYPD
PIVLMKSARN
241       SCWSRDAEYG LYSIYQGGLF ELKKNDRIFV SVTNEHLMDL DQEASFFGAF LIN

SEQ ID NO:4        Flag-TNC-hTRAIL aa95-281.PRO
KEYWORD     PROTEIN

ORIGIN
1          MNFGFSLIFL   VLVLKGVQCE   VKLVPRGSDY   KDDDDKDIAC   GCAAAPDIKD
LLSRLEELEG
61         LVSSLREQGT   GGGSGGRGEF   TSEETISTVQ   EKQQNISPLV   RERGPQRVAA
HITGTRGRSN
121        TLSSPNSKNE   KALGRKINSW   ESSRSGHSFL   SNLHLRNGEL   VIHEKGFYYI
YSQTYFRFQE
181        EIKENTKNDK   QMVQYIYKYT   SYPDPILLMK   SARNSCWSKD   AEYGLYSIYQ
GGIFELKEND
241       RIFVSVTNEH LIDMDHEASF FGAFLVG

SEQ ID NO:5 FLAG-hTNC-GSS-hTRAIL_Thr95_Gly281.pro
KEYWORD PROTEIN
ORIGIN
1 MNFGFSLIFL VLVLKGVQCD YKDDDDKACG CAAAPDVKEL LSRLEELENL
VSSLREQGSS
61 GSSGSSGSGT SEETISTVQE KQQNISPLVR ERGPQRVAAH ITGTRGRSNT
LSSPNSKNEK

121 ALGRKINSWE SSRSGHSFLS NLHLRNGELV IHEKGFYYIY SQTYFRFQEE
IKENTKNDKQ
181 

241 

SEQ ID NO:6          FLAG-hTNC-GSS-hTRAIL_Glu116_Gly281.pro
KEYWORD     PROTEIN
ORIGIN
1          MNFGFSLIFL   VLVLKGVQCD   YKDDDDKACG   CAAAPDVKEL   LSRLEELENL
VSSLREQGSS
61         GSSGSSGSGE   RGPQRVAAHI   TGTRGRSNTL   SSPNSKNEKA   LGRKINSWES
SRSGHSFLSN
121        LHLRNGELVI   HEKGFYYIYS   QTYFRFQEEI   KENTKNDKQM   VQYIYKYTSY
PDPILLMKSA
181        RNSCWSKDAE   YGLYSIYQGG   IFELKENDRI   FVSVTNEHLI   DMDHEASFFG
AFLVG

SEQ ID NO:7          FLAG-hTNC-GSS-hTRAIL_Gly118_Gly281.PRO
KEYWORD     PROTEIN
ORIGIN
1          MNFGFSLIFL  VLVLKGVQCD  YKDDDDKACG  CAAAPDVKEL  LSRLEELENL
VSSLREQGSS
61         GSSGSSGSGP  QRVAAHITGT  RGRSNTLSSP  NSKNEKALGR  KINSWESSRS
GHSFLSNLHL
121        RNGELVIHEK  GFYYIYSQTY  FRFQEEIKEN  TKNDKQMVQY  IYKYTSYPDP
ILLMKSARNS
181      CWSKDAEYGL YSIYQGGIFE LKENDRIFVS VTNEHLIDMD HEASFFGAFL VG

SEQ ID NO:8 FLAG-hTNC-GSS-hTRAIL_Gln120_Gly281.pro
KEYWORD PROTEIN
ORIGIN
1 MNFGFSLIFL VLVLKGVQCD YKDDDDKACG CAAAPDVKEL LSRLEELENL
VSSLREQGSS
61 GSSGSSGSGQ RVAAHITGTR GRSNTLSSPN SKNEKALGRK INSWESSRSG
HSFLSNLHLR
121 NGELVIHEKG FYYIYSQTYF RFQEEIKENT KNDKQMVQYI YKYTSYPDPI
LLMKSARNSC

181 WSKDAEYGLY SIYQGGIFEL KENDRIFVSV TNEHLIDMDH EASFFGAFLV G

SEQ ID NO: 17          SP1-hTRAIL_Glu116_Gly281_GSS_hTNC.pro
KEYWORD     PROTEIN
ORIGIN
1          MNFGFSLIFL VLVLKGVQCE RGPQRVAAHI TGTRGRSNTL SSPNSKNEKA
LGRKINSWES
61         SRSGHSFLSN LHLRNGELVI HEKGFYYIYS QTYFRFQEEI KENTKNDKQM
VQYIYKYTSY
121        PDPILLMKSA RNSCWSKDAE YGLYSIYQGG IFELKENDRI FVSVTNEHLI
DMDHEASFFG
181        AFLVGSSGSS GSSGSGACGC AAAPDVKELL SRLEELENLV SSLREQ

SEQ ID NO: 18          SP3-hTRAIL_Glu116_Gly281_GSS_hTNC.pro
KEYWORD      PROTEIN
ORIGIN
1          METDTLLLWV LLLWVPAGNG ERGPQRVAAH ITGTRGRSNT LSSPNSKNEK
ALGRKINSWE
61         SSRSGHSFLS NLHLRNGELV IHEKGFYYIY SQTYFRFQEE IKENTKNDKQ
MVQYIYKYTS
121        YPDPILLMKS ARNSCWSKDA EYGLYSIYQG GIFELKENDR IFVSVTNEHL
IDMDHEASFF
181        GAFLVGSSGS SGSSGSGACG CAAAPDVKEL LSRLEELENL VSSLREQ

### Examples

### 1. Material and Methods

### Plasmids and cell lines

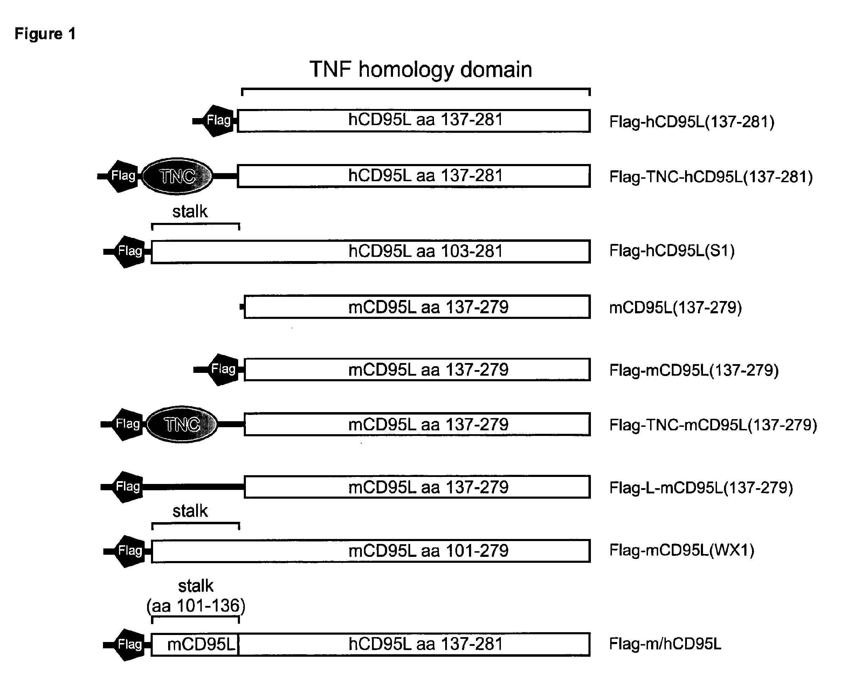

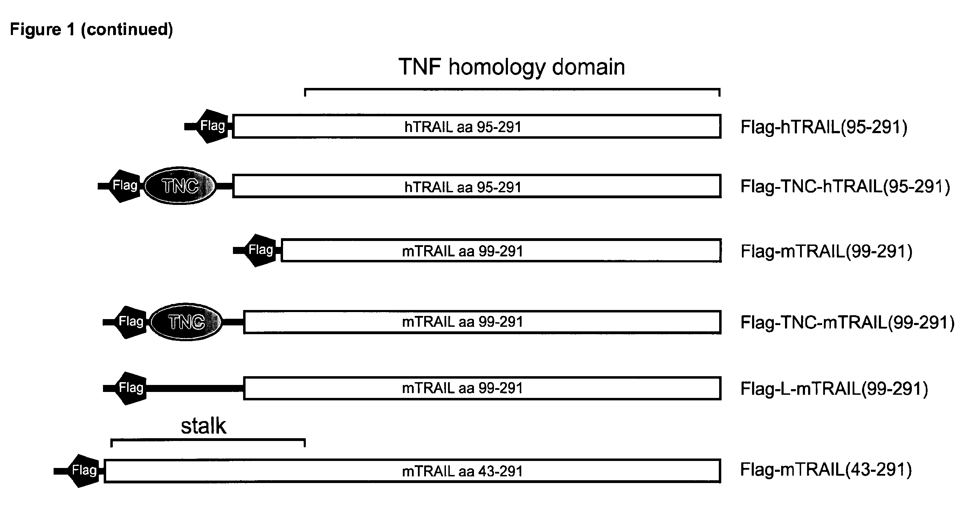

All expression plasmids encoding the proteins summarized in figure 1 were constructed based on the PS435 plasmid which is a modification of pCR3 (Invitrogen, Karlsruhe, Germany) encoding amino-terminally Flag-tagged TRAILR2 with an immunglobulin signal peptide.²¹ To obtain the pCR3 derived expression plasmids encoding the various TRAIL and CD95L fusion proteins, the indicated domains of human TRAIL (U37518; SEQ ID NO:14), murine TRAIL (NM_009425; SEQ ID NO:15), human CD95L (U11821; SEQ ID NO:12) and murine CD95L (NM_010177; SEQ ID NO:13) were amplified with primers having 5' overhangs containing a EcoR1 site and 3' overhangs containing a Xba1 or Xho1 site and used to replace the TRAILR2 part of PS435. To obtain expression plasmids encoding the TNC counterparts of the resulting Flag-tagged TRAIL and CD95L variants, the Flag tag encoding DNA fragment was removed using the flanking BamH1 and EcoR1 sites and replaced by a corresponding amplicon encoding the Flag tag and aa 110 to 139 of chicken tenascin-C (M23121). To obtain the expression plasmids, encoding Flag-L-mCD95L(137-279) and Flag-L-mTRAIL(99-291), the cDNA part encoding the TNC domain was replaced by a linker encoding a similar number of amino acids. MBL2-mCD95 cells are a kind .gift of J.P Medema (Academic Medical Center, Amsterdam, Netherlands) and have been described elsewhere.²²

### Production and purification of recombinant proteins

The different plasmids encoding the various TRAIL and CD95L variants were transfected in HEK293 by electroporation. 40x10⁶ HEK293 cells suspended in 1 ml RPMI supplemented with 10% FCS were electroporated (4 mm cuvette, 250V, 1800 µF, maximal resistance) with 30 µg of plasmid DNA. Cells were recovered overnight in 15 cm dishes with RPMI/10% FCS. Next day, medium was changed with low serum medium (0.5% FCS) and supernatants were collected after additional 3-4 days. Supernatants were concentrated by dialysis on PEG35000 and the recombinant proteins were purified by affinity chromatography using an anti-Flag mAb M2 agarose column (Sigma). Bound proteins were eluted with 100 µg/ml Flag peptide (Sigma) and dialysed against PBS to remove the majority of the Flag peptide. Purified proteins were quantified by BCA (Pierce) and/or by comparison with a flag-tagged protein standard by Western Blotting. Concentrations of nontagged variants of murine TRAIL and CD95L were determined by Western Blot comparison using rat anti-mTRAIL mAb N2B2, a kind gift of Prof. Yagita (Juntendo University School of Medicine, Tokyo, Japan), and goat anti-hCD95L IgG (RD Systems, Wiesbaden, Germany) mAbs samples with their Flag-tagged counterparts at know concentrations. Fc-hTRAIL and Fc-hCD95L are fusion proteins containing the constant region of human IgG1 and the THD of hTRAIL and hCD95L, respectively. Both proteins were kindly prepared by Victoria Schäfer (University of Wuerzburg, Germany).

### Flow cytometry with recombinant TRAIL and CD95L variants

Cells were incubated for 30 min at 4 °C with the indicated concentration of the various variants of CD95L or TRAIL. After washing the cells three times with PBS containing 0.5% bovine serum albumin (BSA, Sigma) cells were incubated with 5 µg/ml anti-Flag mAb M2 (Sigma) for 30 min at 4 °C. After washing three times with PBS/BSA the cell bound fusion protein-antibody complexes were detected using PE-conjugated goat anti-mouse IgG (whole molecule) IgG.

### Chemical crosslinking with BS3

The various variants of CD95L or TRAIL (200 ng) were incubated with varying concentrations of the chemical crosslinker bis(sulfosuccinimidyl) suberate (BS³, Pierce) for 30 min on ice in a total volume of 40 µl. The crosslinking reaction was stopped by quenching with 2 µl 1 M Tris/HCl pH 7.5. Finally, the crosslinked proteins were separated by SDS-PAGE and analyzed by anti-Flag Western Blot.

### Vitality Assays

The various cell lines (KB, L929, MBL2-CD95: 20 x 10³/well; Jurkat 50 x 10³/well) were cultured in 100 µl culture medium in 96-well plates: Next day, cells were treated in triplicates with the indicated concentrations of the various variants of CD95L or TRAIL in the presence and absence of the crosslinking 0.5 µg/ml anti-Flag mAb M2. Holler et al. have previously shown that anti-Flag-induced multimerization becomes suboptimal at higher ligand concentrations⁷. In our experiments this becomes evident especially when TRAIL variants were used. We therefore used in same experiments with TRAIL proteins 3 µg/ml of anti-Flag M2 for crosslinking to induce apoptosis. After 20 h, cell viability was determined by crystal violet staining (KB, L929, MBL2-CD95) or the MTT assay (Jurkat). To sensitize KB and L929 cells for induction of cell death, these cells were stimulated in the presence of 2.5 µg/ml cycloheximide (Sigma).

### Silver staining

Approximately 250 ng of the affinity purified proteins were separated by 16% SDS-PAGE using reducing and non reducing Laemmli sample buffer. After incubation twice with fixer (40% methanol, 12% acetic acid) for 30 min and washing twice with 30% ethanol, gels were treated with 0.02% w/v sodium thiosulfate for 1 min. After three times washing for sec in distilled H₂0 and incubation for 20 min in 0.2% w/v silver nitrate solution freshly supplemented with formaldehyde (1:333), gels were washed again in ddH₂0 (three times for 1 min) and were finally developed with 6% w/v sodium carbonate freshly supplemented with formaldehyde (1:500) and 0.0004% w/v sodium thiosulfate. When the desired signal intensity was achieved the developer was discarded and the reaction was stopped with 5% acetic acid.

### Gel filtration

Protein samples were applied either to a BioSep-SEC-S2000 (300 x 7.8) column or to a BioSep-SEC-S3000 (300 x 7.8) column (Phenomenex, Aschaffenburg, Germany) equilibrated in PBS and eluted at a flow rate of 0.5 ml/min.

### DISC analysis

DISC analysis with Flag-TNC-hTRAIL(95-281) and Flag-hTRAIL(95-281) were performed as recently described elsewhere.²³ Antibodies used to detect FADD, FLIP, TRAILR1 and caspase-8 were purchased from BD (Heidelberg, Germany; FADD) and Axxora (Grünberg, Germany: TRAILR1 and FLIP) or was a kind gift of K. Schulze-Osthoff (University of Düsseldorf, Germany; anti-caspase-8 mAb).

### Surface plasmon resonance analysis of CD95L-CD95 interaction

Dissociation constants (KD) for the binding of Flag-hCD95L(137-281), Flag-mCD95L(137-279) and Flag-TNC-mCD95L(137-279) to an immobilized fusion protein of hlgG1 and the extracellular domain of human CD95 (Fc-CD95) were calculated from surface plasmon resonance measurements (BlAcore 2000, Pharmacia). Fc-CD95 was biotinylated and coupled with 1 µg/ml to the surface of a streptavidin coated sensor chip (Pharmacia) as described elsewhere.²⁴ Sensorgrams were recorded for binding of the various ligands at concentrations ranging from 2 (hCD95L) or 5 nM (mCD95L variants) to 1000 and 5000 nM, respectively, The kinetic constants (kd and ka) were determined using a 1:1 binding model and the software supplied by the manufacturer and were used to calculate the dissociation constant.

### 2. Results and Discussion

### Soluble variants of mCD95L and mTRAIL are practically inactive even after crosslinking

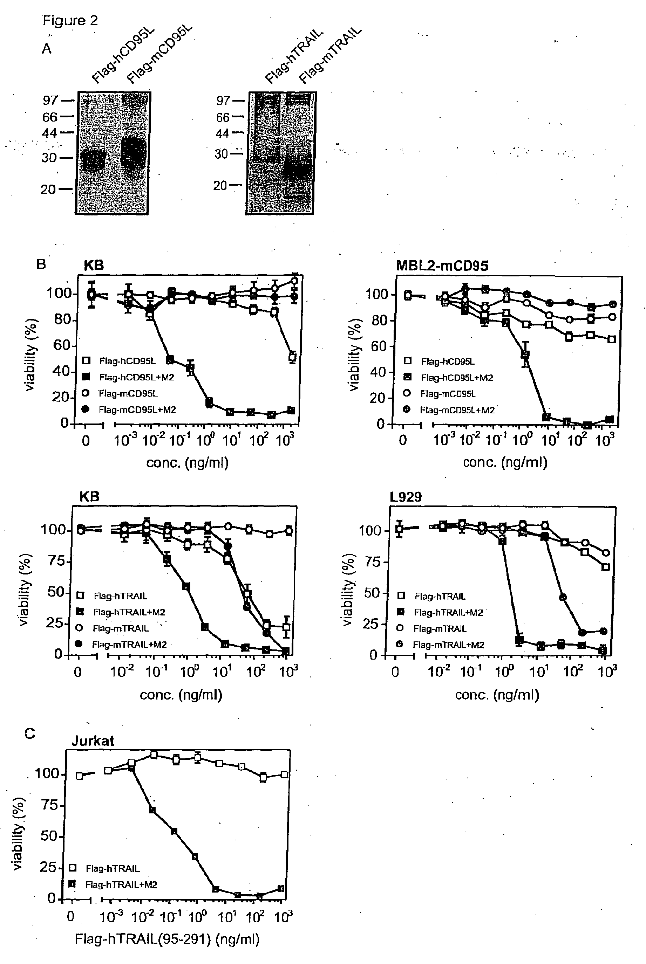

Amino-terminally Flag-tagged variants of human and murine CD95L and TRAIL containing the THD (aa 137-281 of hCD95L, aa 137-279 of mCD95L, aa 95-281 of hTRAIL, aa 99-291 of mTRAIL) were transiently produced in Hek293 cells and purified from the supernatants by affinity chromatography on anti-Flag mAb M2 agarose (Figure 1 and 2a). Both Flag-hCD95L(137-281) and Flag-mCD95L(137-279) migrated in SDS-PAGE analysis as intense doublets with deduced molecular masses between 24 and 30 kDa. In addition, a minor fraction of both proteins also migrated at 21 kDa (Figure 2a). These values significantly exceed the calculated mass of 17 kDa for both Flag-hCD95L(137-281) and Flag-mCD95L(137-279). The heterogeneity and discrepancy between calculated and observed molecular mass is in good accordance with the fact that CD95L can be glycosylated at 3 asparagine residues in the THD, since one N-linked carbohydrate accounts for 2-3 kDa by SDS-PAGE.¹² Flag-hCD95L(137-281) poorly killed human KB cells and murine MBL2-CD95 transfectants cells (ED₅₀: > 1000 ng/ml), but readily killed both cell lines after secondary crosslinking via its Flag-tag (ED₅₀: 0.1 - 1 ng/ml). In contrast, the corresponding Flag-tagged mCD95L (137-279) variant did practically induce no cell death neither in KB nor in MBL2-CD95 cells despite crosslinking (Figure 2b). A related activity pattern was observed with human and murine TRAIL. Both molecules induced less than 25% killing on L929 cells, even at concentrations of 1000 ng per ml. While Flag-hTRAIL(95-281) did already significantly induce cell death in these cells with 1 ng/ml after secondary' crosslinking via the Flag-tag contained in the molecule, Flag-mTRAIL(99-291) was approximately 50fold less active after secondary aggregation (Figure 2b). Further, Flag-hTRAIL (95-281) killed KB cells without crosslinking, starting at concentrations of 10 ng/ml, while non-crosslinked Flag-mTRAIL(99-291) showed no activity at all up to 1000 ng/ml (Figure 2b). Crosslinking with anti-Flag further enhanced the activity of the hTRAIL variant and also allow killing by the murine counterpart at higher concentrations (Figure 2b). The differential effect of noncrosslinked human TRAIL on KB and L929 cells can be explained by earlier findings demonstrating that TRAILR2 is much more better activated by secondary crosslinked trimers of soluble TRAIL than by non-crosslinked molecules, whereas TRAILR1 is stimulated with the same efficiency by crosslinked and non-crosslinked TRAIL.^{5,13,14} Thus, in KB cells that express TRAILR1 and TRAILR2, non-crosslinked TRAIL trimers already significantly induce cell death via the TRAILR1, whereas TRAILR1-mediated cell death is not relevant in the murine L929 cell line, because in mice only a homologue of human TRAILR2 does exist.^{15,16} In fact, in human Jurkat cells, which express only TRAILR2, non-crosslinked Flag-hTRAIL(95-281) is also practically inactive (Figure 2c).

### The THD of mCD95L and mTRAIL is not sufficient to allow receptor binding

Next, we analyzed binding of the various recombinant ligands to cells using FACS analysis.

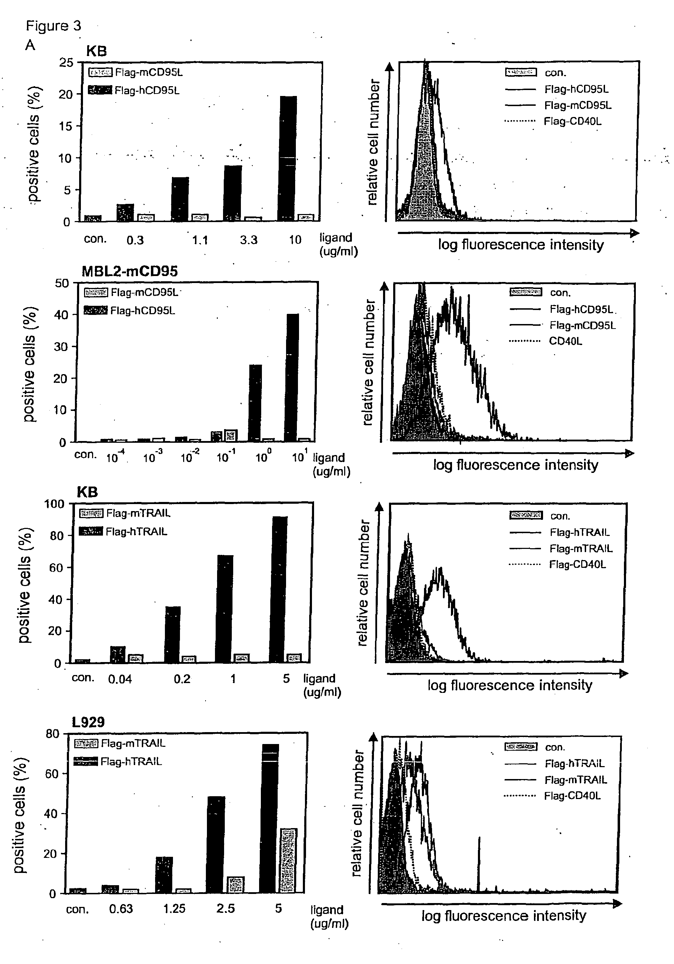

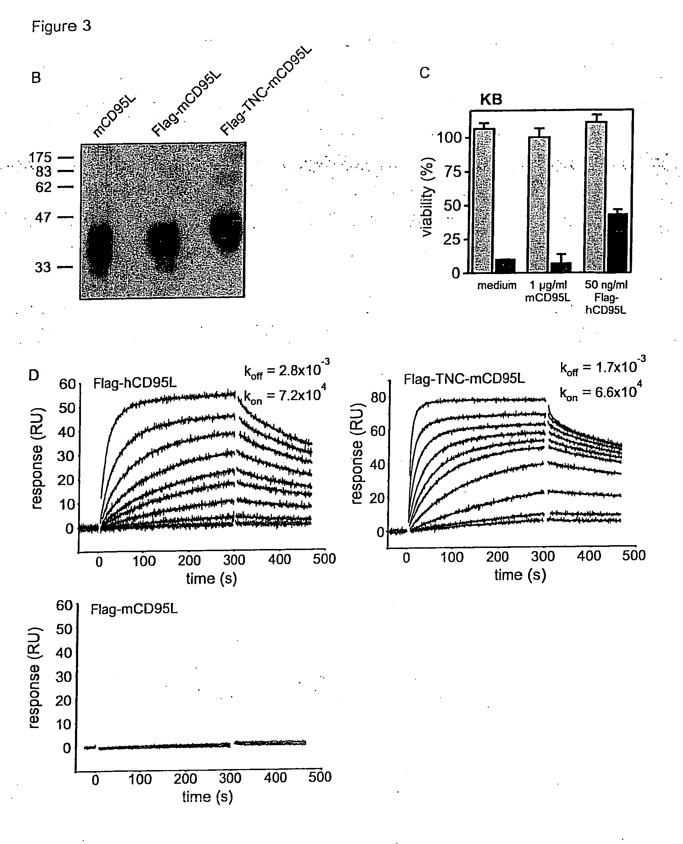

KB, MBL2-CD95 and L929 cells were incubated with increasing concentrations of the TRAIL and CD95L variants on ice and after removal of unbound molecules, cell associated ligands were detected using anti-Flag mAb M2 and a PE-labelled secondary antibody. Significant binding of Flag-hCD95L(137-281) to human and murine CD95 was observed down to concentrations of 0.3 and 0.1 µg/ml and significant binding of Flag-hTRAIL (95-281) to human and murine TRAIL receptors was observed starting with concentrations of 40 ng/ml and 600 ng/ml, respectively. In contrast, murine CD95L showed no binding even when concentrations up to 5 or 10 µg/ml were used and murine TRAIL exerted only weak binding on L929 cells and no binding on KB cells (Figure 3a). Binding of the murine ligands were also not significantly changed when they were preincubated with the M2 antibody prior to FACS staining (data not shown). To exclude the possibility that the Flag-tag, which is highly charged, specifically prevented receptor binding of Flag tagged proteins, we also analyzed a non-tagged variant of mCD95L (Figure 3b). Due to the lack of the Flag epitope, we were unable to analyze receptor binding of mCD95L(137-279) directly by FACS. Instead, we performed a competition assay, in which the capacity of the non-tagged ligand to protect against cell death induction was determined using a highly active hexameric fusion protein of hCD95L (Fc-hCD95L) and the Fc domain of human IgG1 (see ref. 7) was determined.

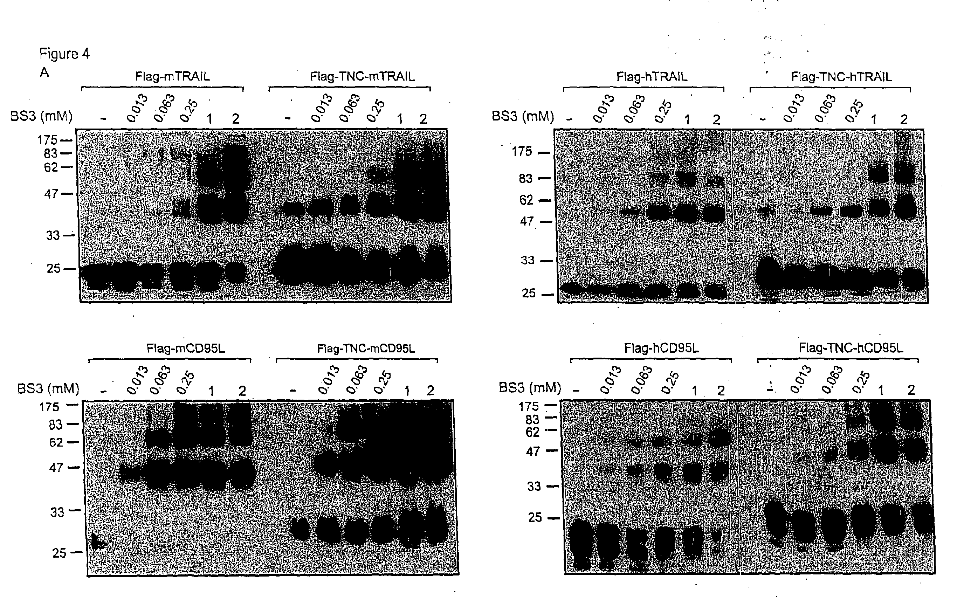

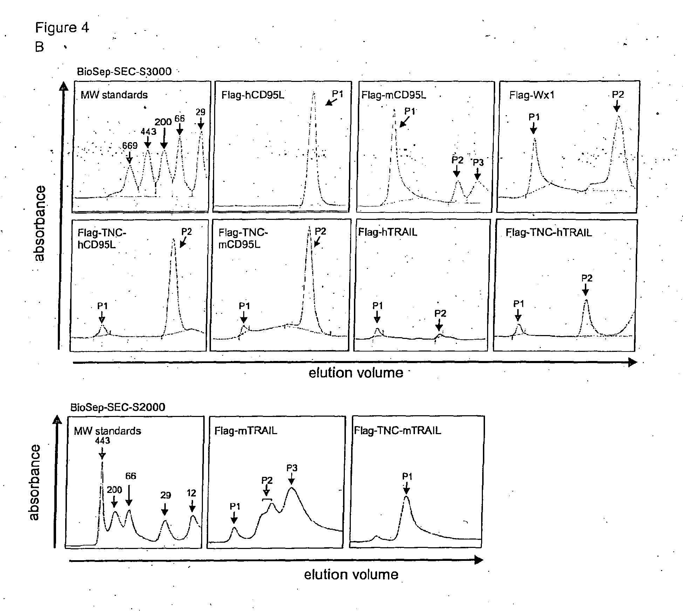

While preincubation of KB cells with 50 ng/ml Flag-hCD95L(137-281) was sufficient to rescue about half of the cells from Fc-hCD95L-induced cell death, the non-tagged mCD95L(137-279) failed even at high concentrations (1 µg/ml) to show a protective effect (Figure 3e). We also performed surface plasmon resonance analysis, using an immobilized chimeric protein consisting of the extracellular domain of human CD95 fused to the constant region of human IgG1 and Flag-hCD95L(137-281), Flag-mCD95L(137-279) or Flag-TNC-mCD95L(137-279) in solution. For Flag-TNC-mCD95L(137-279) and Flag-hCD95L(137-281) we obtained dissociation constants of 26 and 39 nM. With Flag-mCD95L(137-279) non specific binding was observed even at concentration of 2000 nM (Figure 3d). Together, our data indicate that the receptor binding of both murine non-tagged ligand variants is negligible. A possible explanation for the inability/poor ability of Flag-mCD95L(137-279) and Flag-mTRAIL(99-291) to interact with their corresponding receptors is that the THDs of these molecules, determined by sequence homology with their human counterparts, are not sufficient to drive ligand trimerization. We therefore investigated the various recombinant ligands by chemical crosslinking and gel filtration. Treatment of Flag-hCD95L(137-281) and Flag-hTRAIL(95-281) with increasing concentrations of the homobifunctional crosslinker BS3 allowed, as expected, the identification of dimers and trimers (Figure 4a). Notably, crosslinking of the murine ligands lead to formation of dimeric and trimeric adducts with a similar BS3 dose dependency as observed with their human counterparts (Figure 4a). Thus, there was no evidence from the crosslinking experiments for a differential capability of murine and human ligands to organize into trimers. In gel filtration experiments Flag-hCD95L(137-281) was eluted in one symmetric peak which corresponds in size to a molecular mass of about 72 kDa (Table 1, Figure 4b) in good agreement with a trimeric organization of the glycosylated molecule in solution. The hCD95L preparation obtained after gel filtration did still interact with CD95 and showed normal activity after secondary crosslinking (data not shown). Flag-mCD95L(137-279) was eluted in a high molecular weight peak, a second peak with an elution volume comparable to that of Flag-hCD95L(137-281) trimers (85 kDa) and a third peak corresponding in size to a monomer (31 kDa), (Figure 4b). As expected Flag-mCD95L(137-279) derived of each of these peaks was inactive even after crosslinking with the anti-Flag mAb M2 (Table 1). Thus, the high molecular weight Flag-mCD95L(137-279) fractions obviously represented misfolded inactive ligand aggregates rather than activated secondary aggregates of latently active ligand trimers. A similar picture emerged when the various TRAIL variants were analyzed by gel filtration. In all cases there was a peak corresponding in size to MWs of 75 to 125 kDa and thus to a trimeric organization of the corresponding molecules (Table 1, Figure 4b). In addition, we did also observe a second high molecular weight peak containing inactive trimeric death ligands with TRAIL aggregates (Table 1). Flag-mTRAIL(99-291) was further found in an additional peak, corresponding in size roughly to a monomer.

### Covalent stabilization of the trimeric structure of mCD95L and mTRAIL restores receptor binding and crosslinking dependent activity

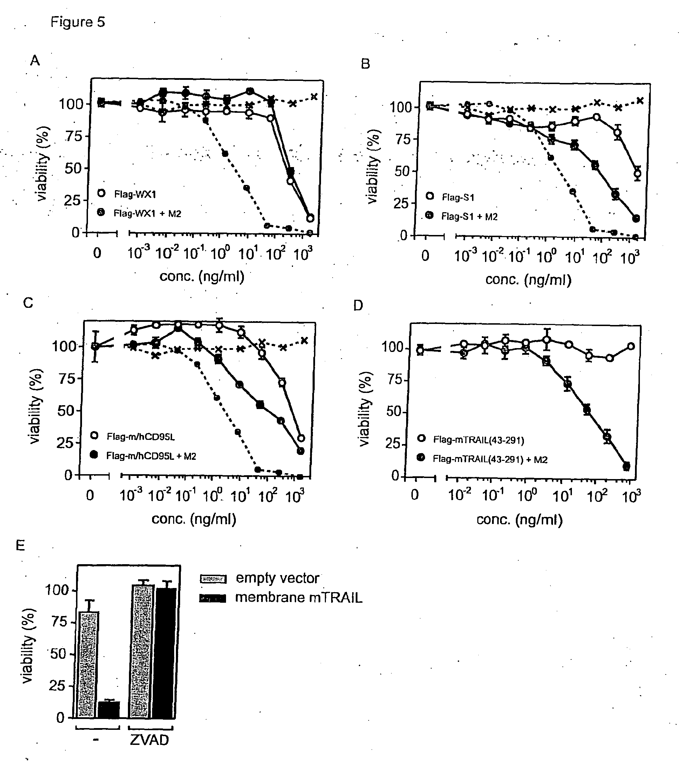

So far our data demonstrated that the trimerization of the THD alone is not necessarily sufficient to ensure proper receptor binding. This suggests that especially in case of mCD95L and mTRAIL additional structural cues located outside of the THD are required to enable these molecules to bind their cognate receptors. In this regard, the THDs of CD95L and TRAIL are separated from their transmembrane domains by a stalk region. In fact, the group of Nagata has shown that a variant of mCD95L (named WX1) comprising aa 101-279 and thus containing the stalk region is biologically active.¹⁷ Noteworthy, Flag-mCD95L(WX1) is already active without secondary crosslinking and organizes to about 50% in oligomers instead of trimers (Table 1; Figure 1, 4 and 5a). Moreover, secondary crosslinking of the Flag-tagged variant of WX1 did not further enhance the activity of the molecule (Figure 5a).

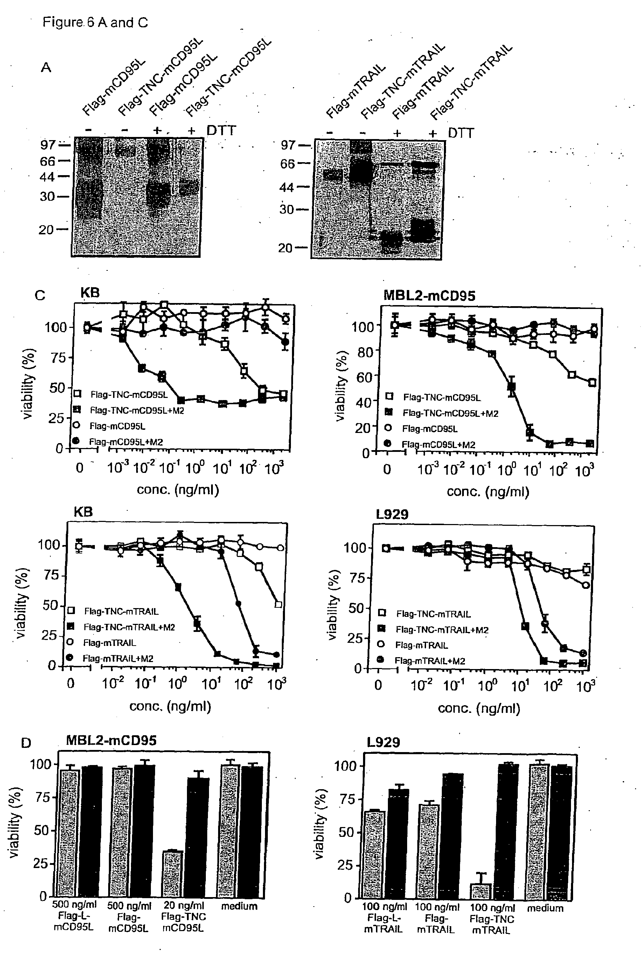

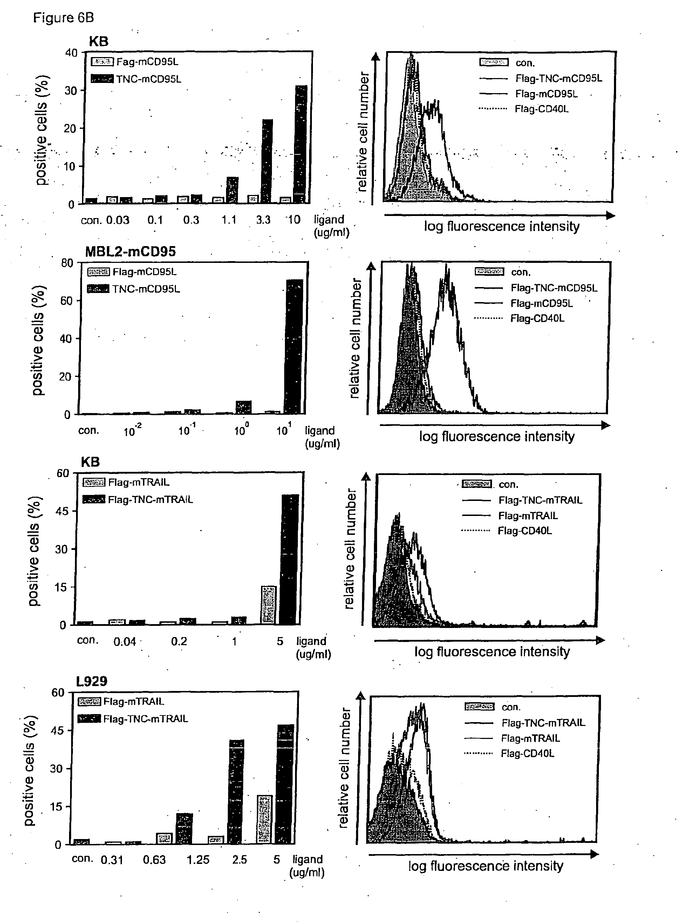

The Flag-tagged human counterpart of the WX1 variant (named S1) showed also significant activity which could be further moderately increased by secondary crosslinking (Table 1; Figure 1 and 5b). The specific activity of crosslinked Flag-hCD95L(S1) was roughly 100 fold lower compared to crosslinked Flag-hCD95L(137-281) (Figure 5b). A chimeric ligand of the stalk region of mCD95L and the THD of hCD95L showed also a significant crosslinking independent capability to stimulate CD95 (Figure 5c). Flag-mTRAIL(43-291), a soluble mTRAIL variant containing the stalk region, showed a significant, but in comparison to Flag-hTRAIL(114-291) poor capability to induce cell death (Figure 5d). Of course, membrane mTRAIL was also able to stimulate TRAIL receptor signaling (Figure 5e). Together, these data suggest that the stalk region contained-in some members of the TNF family, e.g. murine CD95L, has a ligand specific capability to facilitate the formation of secondary aggregated trimers. The different bioactivity of soluble ligand variants with and without stalk region might be explained by the concept that amino acids located outside the THD are required to make contact to their corresponding receptors, but it appeared also possible that the THD of mTRAIL and mCD95L already contained latently all structural information required for receptor binding, but needed in addition spatial fixation and stabilization of their trimeric structure. In fact, a difference between soluble: trimeric TRAIL and CD95L and their highly active membrane-bound counterparts is that the amino terminus of the THD in the soluble molecules is practically free, whereas in the membrane bound ligands the amino-terminal part of the THD is fixed by the transmembrane domain and/or the stalk region. To elucidate whether the improper receptor binding of stalk-less mCD95L and mTRAIL trimers was related to insufficient stability and spatial fixation of the THD of these molecules, we generated fusion proteins of these ligands in which the flexibility of the amino termini was constrained by fusion to a small protein domain from chicken tenascin-C (TNC) forming covalently bound compact trimers (Figure 1).¹⁸ The tenascin-C trimerization domain was chosen due to its small size of about only 30 aa.¹⁸ Anti-Flag affinity purified TNC-Flag-mTRAIL(99-291) and Flag-TNC-mCD95L(137-279) migrated both in non-reducing SDS-PAGE analysis as trimers and showed in gel filtration analysis a comparable elution volume than the corresponding molecules without TNC domain (Figure 4a, 6a and Table 1). These data showed firstly that the TNC part within the fusion proteins is still able to form disulfide bonds and indicated secondly that the serial arrangement of the trimerization domains of TNC and TNF ligands still results in formation of trimers without evidence for a significant formation of high molecule weight aggregates. In further accordance with a trimeric organization of the TNC fusion proteins and a lack of secondary aggregation, we detected after chemical crosslinking with increasing concentrations of the homobifunctional crosslinker BS3 preferentially dimers and trimers (Figure 4a). Thus, the THD of mCD95L and mTRAIL, irrespectively of having a TNC domain or not, organizes into trimers. However, with respect to their functional properties the molecules behaved quite differently. As shown already in figure 3 Flag-mCD95L(137-279) and Flag-mTRAIL(99-291) did not or only poorly interact with their cognate cellular receptors, but the TNC fusion proteins showed effective binding in FACS analysis and resembled in this regard their human counterparts (Figure 6b and 3a). Moreover, the murine TNC fusion proteins are practically inactive despite receptor binding, but stimulated CD95 and TRAIL death receptor signaling after secondary crosslinking with high efficiency (Figure 6c). To exclude the possibility that the improved receptor binding of the TNC variants is caused by the more distant position of the Flag epitope in TNC-mCD95L(137-281) and Flag-TNC-mTRAIL(99-291) relative to the' THD domain, rather than by the presence of the TNC domain, we generated ligand variants (Flag-L-mCD95L(137-279) and Flag-L-mTRAIL(99-291)) in which the Flag epitope was separated from the THD of the molecules via a linker corresponding in length to the TNC domain. We found that these ligand variants showed no (Flag-L-mCD95L(137-279)) or only weak activity (Flag-L-mTRAIL(99-291)) without crosslinking. This suggests that the position of the Flag-tag relative to the THD domain of murine ligands has no major relevance for receptor binding of these molecules (Figure 6d). As the TNC domain is sufficient to recover the receptor binding capacity of the THD of mCD95L and mTRAIL, it is unlikely that specific amino acids located outside the THD are involved in direct interaction with the cognate receptors. Thus, spatial fixation of the amino-terminal part of the THD appears in some members of the TNF ligand family necessary to ensure proper receptor binding, e.g. by stabilizing the THD structure against structural disturbance related to receptor binding. So, the stalk regions of CD95L and TRAIL can fulfil two functions: i) stabilization of the THD, a function which can also be achieved by the TNC domain and ii) secondary aggregation of THD trimers enabling CD95 activation, a function which is not achieved by the TNC domain.

However, the functions fulfilled by the stalk region are dependent on the ligand regarded and might be realized in concert with the transmembrane domain. E.g. the stalk region of hCD95L is dispensable for receptor binding, but mediates to some extend secondary aggregation and the stalk region of mTRAIL seems to stabilize the THD comprising trimer, but does not overcome the need of secondary aggregation for receptor activation.

### Fusion proteins of the trimerization domain of TNC and the THD of hTRAIL or hCD95L display higher activity than their TNC less counterparts

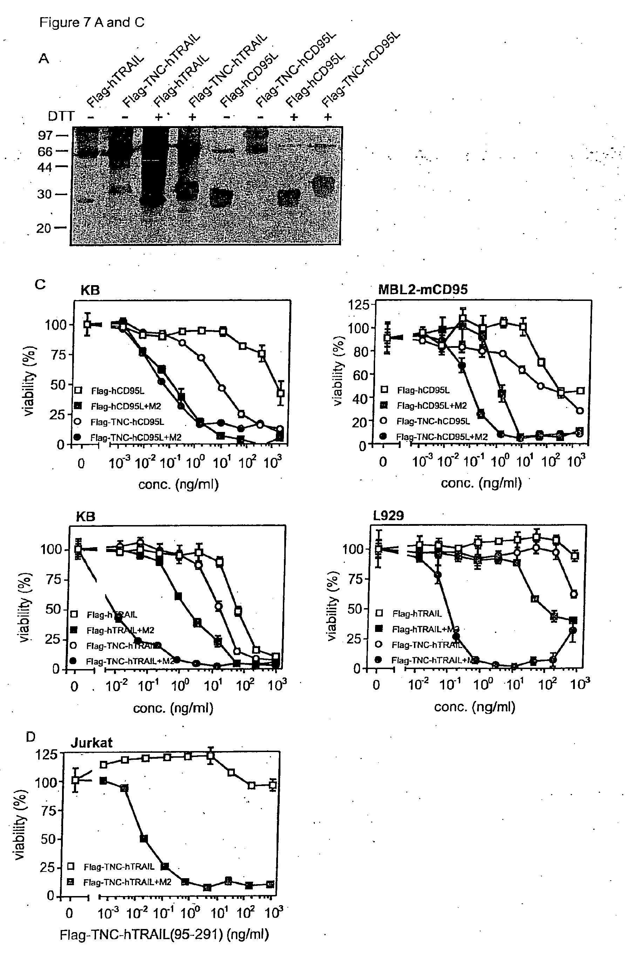

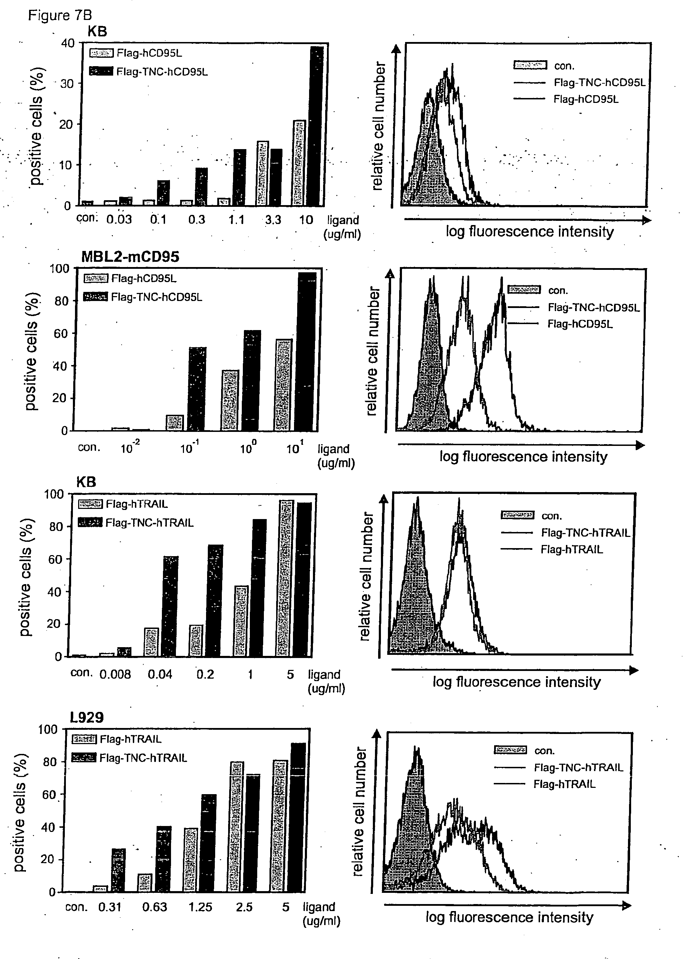

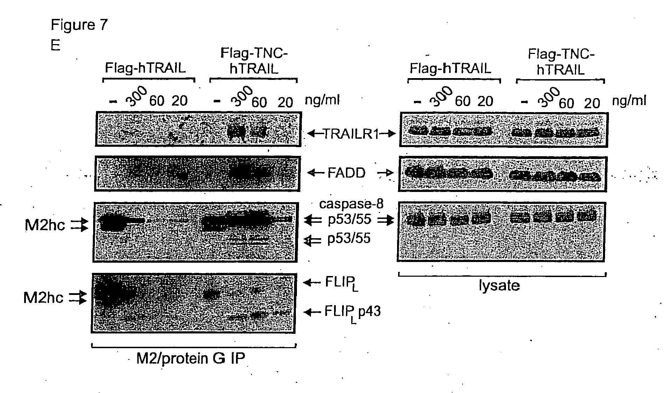

To test whether spatial fixation of the THD also improves receptor binding and activity of soluble variants of hTRAIL (Flag-hTRAIL(95-281) and hCD95L (Flag-hCD95L(137-281), we generated and analysed the corresponding TNC fusion proteins (Flag-TNChCD95L(137-281) and Flag-TNC-hTRAIL(95-281), Figure 7a). Crosslinking experiments with BS3 and gel filtration analysis indicated that Flag-TNC-hCD95L(137-281) and Flag-TNC-hTRAIL(95-281) assemble into trimers (Figure 4 and Table 1). FACS analysis further showed that both TNC fusion proteins were somewhat more efficient (3 - 10fold) than their TNC-less counterparts in receptor binding (Figure 7b). Flag-TNC-hCD95L(137-281) showed a 5 to 30 fold lower ED₅₀ value for induction of cell death, compared to Flag-hCD95L(137-281). Non-crosslinked Flag-TNC-hTRAIL(95-281) showed regularly approximately 5fold lower ED₅₀ values for apoptosis induction than its TNC-less counterpart. With respect to the capability to induce cell death after crosslinking, however, the differences were much more pronounced with100 to 200fold lower ED₅₀ values for Flag-TNC-hTRAIL(95-281), (Figure 7c). The need for ligand crosslinking to activate human TRAILR2 is also required for Flag-TNC-hTRAIL(95-281), as apoptosis induction in Jurkat cells expressing, which express no TRAILR1, is still strictly dependent on crosslinking (figure 7d). In accordance with the higher activity of Flag-TNC-hTRAIL(95-281), DISC formation was also more efficient with this variant, compared with Flag-hTRAIL(95-281). (Figure 7e). It is intriguing that Flag-TNC-hTRAIL(95-281) showed a comparably low increase in receptor binding and a moderate increase in apoptosis induction (as non-crosslinked molecule) compared to Flag-hTRAIL(95-281), but a marked increase in cell death induction under crosslinked conditions. The differential relevance of the TNC domain for different properties of Flag-TNC-hTRAIL(95-281) and Flag-hTRAIL(95-281) is not clear yet, but could be related to two obvious points.

Firstly, the importance of the TNC domain for receptor binding could be receptor specific. As KB as well as L929 cells express not only the TRAIL death receptors, but also the TRAIL decoy receptors; it can not be ruled out that latter binds quite well to the TNC-less TRAIL variant masking a more pronounced effect on binding to the TRAIL death receptors.

Furthermore, in the human system TRAILR1 and TRAILR2, which are differentially, dependent on ligand crosslinking for activation, are also differentially regulated by TRAILR4.^{19,20} Secondly, the transformation of receptor occupancy by ligand into DISC formation and further to activation of apoptotic caspases could be non-linear and receptor specific.

Taken together, fusion with the TNC domain did not overcome the requirement of human CD95L and human TRAIL for secondary crosslinking to become properly active (Figure 7c,d). This is in agreement with the fact that the TNC domain does not mediate secondary aggregation of ligand trimers. Thus, the introduction of a TNC domain appears to be a broadly applicable option to generate recombinant soluble ligands of the TNF family with superior activity.

### Table 1 Gel filtration analysis of recombinant TRAIL and CD95L variants

20 µl of the various M2 agarose purified proteins were fractionated by gel filtration using a BioSep-SEC-S2000 (300 x 7.8) column (all CD95L variants, Flag-hTRAIL, FlagTNChTRAIL) or a BioSep-SEC-S2000 (300 x 7.8) column (Flag-mTRAIL and Flag-TNC-mTRAIL). The columns were calibrated with thyroglobulin (669 kDa), apoferritin (443 kDa), β-amylase (200 kDa), serum albumin (66 kDa), carbonic anhydrase (29 kDa) and cytochrom c (12.4 kDa). The molecular masses corresponding to the elution volumes of the various peaks were calculated using the linear regression of the logarithm of the molecular masses of the standards against their elution volume. Peaks were collected and analyzed as indicated with respect to the presence of the corresponding ligand by western blot or ELISA to binding and cell death induction in KB or L929 cells. The peak areas were automatically calculated by the FPLC software and indicate the relative amount of protein contained in the peak. To determine the capacity of proteins in the various peaks to induce cell death, the corresponding samples were titrated on CHX sensitized KB cells. The fold dilution sufficient to induced 50% killing was indicated. n.d. not determined. HWA. high molecular weight aggregates.

**Table 1**

| protein | MW kDa | peak area mVxs | FACS binding | activity no M2 | activity + M2 |
|---|---|---|---|---|---|
| | | | | dilution of ED50 | |
| Flag-mCD95L(137-279) | | | | | |
| peak 1 | HWA | 463 | no | no | no |
| peak 2 | 86 | 109 | no | no | no |
| peak 3 | 32 | 224 | no | no | no |
| | | | | | |
| Flag-TNC-mCD95L(137-279) | | | | | |
| peak 1 | HWA | 14 | weak | << 1 | << 1 |
| | | | | | |
| peak 2 | 128 | 380 | yes | << 360 | 12960 |
| | | | | | |
| Flag-hCD95L(137-281) | 72 | 692 | yes | << 60 | 12960 |
| | | | | | |
| Flag-TNC-hCD95L(137-281) | | | | | |
| peak 1 | HWA | 29 | n.d. | << 1 | << 1 |
| peak 2 | 104 | 337 | yes | << 10 | 2160 |
| | | | | | |
| Flag-WX1 | | | | | |
| peak 1 | HWA | 93 | n.d. | n.d. | n.d. |
| peak 2 | 48 | 230 | n.d. | n.d. | n.d. |
| | | | | | |
| Flag-mTRAI L(99-291) | | | | | |
| peak 1 | HWA | 49 | n.d. | n.d. | n.d. |
| peak 2 | 75-90 | 184 | n.d. | << 1 | << 1 |
| peak 3 | 35 | 429 | n.d. | n.d. | n.d. |
| | | | | | |
| Flag-TNC-mTRAIL(99-291) | 85 | 192 | n.d. | active | active |
| | | | | | |
| Flag-hTRAIL(95-291) | | | | | |
| peak 1 | HWA | 12 | n.d | << 1 | << 1 |
| peak 2 | 125 | 2.7 | n.d. | 4 | 100 |
| | | | | | |
| Flag-TNC-hTRAIL(95-291) | | | | | |
| peak 1 | HWA | 18 | n.d | n.d. | n.d. |
| peak 2 | 125 | 109 | n.d. | active | active |

**Table 2**

| **Approved Gene symbol** | **TNFSF-number** | **Synonyms** | **Accession** | **NH2-aa** | **COOH-aa** | **Length** |
|---|---|---|---|---|---|---|
| LTA | TNFSF-1 | LTA | gi\|6806893\|ref\|NP 000586.2\| | Ser59 | Leu205 | 147aa |
| | | | | Thr60 | Leu205 | .146aa |
| TNF | TNFSF-2 | TNF-alpha | gi\|25952111\|ref\|NP 000585.2\| | Asp86 | Leu233 | 148aa |
| LTB | TNFSF-3 | LTB | gi\|4505035\|ref\|NP 002332.1\| | Asp82 | Gly244 | 163aa |
| TNFSF4 | | | | Gly86 | Gly244 | 159aa |
| | TNFSF-4 | OX40L/GP34 | gi\|4507603\|ref\|NP 003317.1\| | Val52 | Leu183 | 132aa |
| | | | | Arg55 | Leu183 | 129aa |
| CD40LG | TNFSF-5 | CD40L | gi\|4557433\|ref\|NP 000065.1\| | Asp117 | Leu262 | 150aa |
| | | | | Glu112 | Leu262 | 145aa |
| FASLG | TNFSF-6 | CD95L/APO- | gi\|4557329\|ref\|NP 000630.1\| | Glu142 | Leu281 | 140aa |
| | | L/FAS-L | | Arg144 | Leu281 | 1388a |
| TNFSF7 | TNFSF-7 | CD27L | gi\|4507605\|ref\|NP 001243.1\| | Glu51 | Pro193 | 143aa |
| | | | | Asp56 | Pro193 | 138aa |
| TNFSF8 | TNFSF-8 | CD30L | gi\|4507607\|ref\|NP 001235.1\| | Lys97 | Asp234 | 138aa |
| | | | | Ser98 | Asp234 | 137aa |
| | | | | Leu102 | Asp234 | 133aa |
| TNFSF9 | TNFSF-9 | 4-1BB/CD137L | gi\|4507609\|ref\|NP 003802.1\| | Asp86 | Glu254 | 169aa |
| TNFSF10 | TNFSF-10 | TRAIL | gi\|4507593\|ref\|NP 003801.1\| | Glu116 | Gly281 | 166aa |
| | | | | Gly118 | Gly281 | 164aa |
| TNFSF11 | TNFSF-11 | TRANCE/RANK L | gi\|4507595\|ref\|NP 003692.1\| | Glu161 | Asp317 | 157aa |
| TNFSF12 | TNFSF-12 | TWEAK/Apo-3 | gi\|4507597\|ref\|NP 003800.1\| | Ala103 | His249 | 147aa |
| | | | | Arg104 | His249 | 146aa |
| | | | | Arg105 | His249 | 145aa |
| TNFSF13 | TNFSF-13 | APRIL/TALL-2/TRDL-1 | gi\|26051248\|ref\|NP 742085.1\| | Lys112 | Leu247 | 136aa |
| TNFSF13 | TNFSF-13 | APRIL/TALL-2/TRDL-1 | gi\|4507599\|ref\|NP 003799.1\| | Lys112 | Leu250 | 139aa |
| TNFSF13B | TNFSF-13B | BAFF/Blys | gi\|5730097\|ref\|NP 006564.1\| | Glu140 | Leu285 | 146aa |
| TNFSF14 | TNFSF-14 | LIGHT | gi\|25952144\|ref\|NP 003798.2\| | Glu91 | Val240 | 150aa |
| TNFSF15 | TNFSF-15 | TL1A/VEGI | gi\|23510445\|ref\|NP 005109.2\| | Asp91 | Leu251 | 161aa |
| | | | | Asp93 | Leu251 | 159aa |
| TNFSF18 | TNFSF-18 | GITRL | gi\|4827034\|ref\|NP 005083.1\| | Glu52 | Ser177 | 126aa |
| EDA | | EDA-A1 | gi\|4503449\|ref\|NP 001390.11 | Glu245 | Ser391 | 147aa |
| EDA | | EDA-A2 | gi\|54112101\|ref\|NP 001005609.1\| | Glu245 | Ser389 | 145aa |

## Claims

1. A fusion protein comprising
(i) a TNF-superfamily cytokine or a receptor binding domain thereof
(ii) a flexible linker element between (i) and (iii), and
(iii) a tenascin trimerization domain with the provision that in case (iii) is located N-terminally of (i) no scFvMO36-selectokine component is located N-terminally of (iii).

2. Fusion protein of claim 1 wherein (i) is selected from CD95L, TRAIL, TNFα, or a receptor binding domain thereof.

3. Fusion protein of claim 2 wherein (i) is CD95L or a receptor binding domain thereof.

4. Fusion protein of claim 3 wherein (i) comprises amino acids 120-281, 137-281, 142-281 or 144-281 of human CD95L (SEQ ID NO:12) or amino acids 137-279 of mouse CD95L (SEQ ID NO:13).

5. Fusion protein of claim 2 wherein (i) is TRAIL or a receptor binding domain thereof.

6. Fusion protein of claim 5 wherein (i) comprises amino acids 95-281, 116-281, 118-281 or 120-281 of human TRAIL (SEQ ID NO:14) or amino acids 99-291 of mouse TRAIL (SEQ ID NO:15).

7. Fusion protein of any one of claims 1-6 wherein (ii) has a length of 3-20 amino acids, particularly a length of 3, 6, 9, 10, 12, 15-or 18 amino acids.

8. Fusion protein of claim 7 wherein (ii) has the amino acid sequence GTGGGSGGRG.

9. Fusion protein of claim 7 wherein (ii) is a glycine/serine linker.

10. Fusion protein of claim 9 wherein (ii) has the amino acid sequence (GSS)ₐ(SSG)_{b}(GSG)_{c} wherein a, b, c is each 0, 1, 2, 3, 4 or 5 or (GSS) ₙGSG, (SGG)ₙGSG or (GSS)ₙ wherein n is 1, 2, 3, 4, or 5.

11. Fusion protein of any one of claims 1-10 wherein (iii) is from chicken or human.

12. Fusion protein of claim 11 wherein (iii) comprises the amino acid sequence ACGCAAAPDVKELLSRLEELENLVSSLREQ (SEQ ID NO:9) or a sequence having an identity of at least 70% thereto.

13. Fusion protein of any one of claims 1-12 which additionally comprises an N-terminal signal peptide domain, which may comprise a protease cleavage site.

14. Fusion protein of claim 13 wherein the N-terminal signal peptide domain comprises the sequence SEQ ID NO:10, SEQ ID NO:11, or SEQ ID NO:16.

15. Fusion protein of any one of claims 1-14 wherein the fusion protein comprises the sequence SEQ ID NO:1, SEQ ID NO:2, SEQ ID NO:3, SEQ ID NO:4, SEQ ID NO:5, SEQ ID NO:6, SEQ ID NO:7 or SEQ ID NO:8.

16. Fusion protein of any one of claims 5-14 wherein (i) comprises a mutant of TRAIL or of a receptor binding domain thereof which binds and/or activates TRAILR1 and/or TRAILR2.

17. Fusion protein of claim 16 wherein the mutant comprises at least one amino acid substitution.

18. Fusion protein of claim 17 wherein the amino acid substitution affects at least one of the following amino acid positions of human TRAIL (SEQ ID NO:14): R130, G160, Y189, R191, Q193, E195, N199, K201, Y213, T214, S215, H264, I266, D267, D269.

19. Fusion protein of claim 18 wherein the amino acid substitution is at least one of the following: R130E, G160M, Y189A, Y189Q, R191 K, Q193S, Q193R, E195R, N199V, N199R, K201R, Y213W, T214R, S215D, H264R, I266L, D267Q, D269H, D269R, or D269K.

20. Fusion protein of any one of claims 1-14 or 16-19 wherein (i) is located N-terminally of (iii).

21. Fusion protein of claim 20 wherein the fusion protein comprises the sequence SEQ ID NO:17 or SEQ ID NO:18.

22. Fusion protein of any one of claims 1-21 wherein the fusion protein further comprises a recognition/purification domain.

23. Fusion protein of claim 22 wherein the recognition/purification domain is located at the N-terminus or at the C-terminus.

24. Fusion protein of claims 22 or 23 wherein the recognition/purification domain is a Flag-tag.

25. Fusion protein of any one of claims 1-24 which additionally comprises a terminal flexible element which may include and/or connect to the recognition/purification domain.

26. Fusion protein of any of claims 1-25 which is present as a trimeric complex or as an oligomer of the trimeric complex.

27. Fusion protein of claim 26 wherein the complex is formed by covalent linkage between three fusion proteins.

28. Fusion protein of claim 27 wherein the covalent linkage consists of disulfide bridges between two conserved cysteines of (iii).

29. Fusion protein of any one of claims 26-28 wherein the complex consists of three identical fusion proteins.

30. A nucleic acid molecule encoding a fusion protein of any one of claims 1-25.

31. Nucleic acid molecule of claim 30 which is operatively linked to an expression control sequence.

32. Nucleic acid molecule of claims 30 or 31 which is located on a vector.

33. A cell transformed or transfected with a nucleic acid molecule of any one of claim 30-32.

34. The cell of claim 33 which is a prokaryotic cell.

35. The cell of claim 33 which is a eukaryotic cell, preferably a mammalian cell and more preferably a human cell.

36. A non-human organism transformed or transfected with a nucleic acid molecule of any one of claim 30-32.

37. A pharmaceutical composition comprising as an active agent a fusion protein of any one of claims 1-29, a nucleic acid molecule of any of claims 30-32, or a cell of any one of claims 33-35.

38. A diagnostic composition comprising as an active agent a fusion protein of any one of claims 1-29, a nucleic acid molecule of any of claims 30-32, or a cell of any one of claims 33-35.

39. A fusion protein of any one of claims 1-29, a nucleic acid molecule of any of claims 30-32, or a cell of any one of claims 33-35 for use in therapy.

40. Use of a fusion protein of any one of claims 1-29, a nucleic acid molecule of any of claims 30-32, or a cell of any one of claims 33-35 for the preparation of a pharmaceutical composition in the prophylaxis and/or treatment of proliferative disorders, particularly disorders caused by, associated with and/or accompanied by dysfunction of TNF cytokines, such as tumors, e.g. solid or lymphatic tumors, infectious diseases, inflammatory diseases, metabolic diseases, autoimmune disorders, e.g. rheumatoid and/or arthritic diseases, degenerative diseases, e.g. neurodegenerative diseases such as multiple sclerosis, apoptosis-associated diseases and transplant rejections.
